# FASCICULE DE BREVET EUROPEEN

(11) **EP 2 182 914 B1**
(45) Date de publication et mention de la délivrance du brevet: **08.11.2017**
(21) Numéro de dépôt: 08827084.8
(22) Date de dépôt: 17.07.2008
(51) Int. Cl.: A61K 8/25, A61K 8/73, A61Q 5/10

(54) **COMPOSITION DE TEINTURE D'OXYDATION DES FIBRES KERATINIQUES COMPRENANT UN ETHER DE CELLULOSE CATIONIQUE, UN METASILICATE ET DES COLORANTS D'OXYDATION, PROCEDE DE TEINTURE D'OXYDATION ET UTILISATION**
ZUSAMMENSETZUNGSOXIDATIONSFÄRBUNG VON KERATINFASERN MIT EINEM KATIONISCHEN CELLULOSEETHER, EINEM METASILIKAT UND OXIDATIONSFÄRBEMITTELN, VERFAHREN ZUR FÄRBUNG UND VERWENDUNGEN DAVON
COMPOSITION OXIDATION COLOURING OF KERATINIC FIBRES, CONTAINING A CATIONIC CELLULOSE ETHER, A METASILICATE AND OXIDATION DYES, METHOD FOR OXIDATION COLOURING AND USES THEREOF

(30) Priorité: 31.07.2007 FR 0756856; 29.08.2007 US 935741 P
(43) Date de publication de la demande: 12.05.2010
(73) Titulaire: L'Oréal, 75008 Paris (FR)
(72) Inventeur: AUDOUSSET, Marie-Pascale, F-92600 Asnieres (FR)
(74) Mandataire: Dossmann, Gérard
(86) Numéro de dépôt international: PCT/FR2008/051339
(87) Numéro de publication internationale: WO 2009/019383

(56) Documents cités:
- EP-A- 1 707 181
- EP-A- 1 747 774
- WO-A-2006/099163
- FR-A- 2 838 337
- DROVETSKAYA T V ET AL: "EFFECTS OF LOW-LEVEL HYDROPHOBIC SUBSTITUTION ON CONDITIONING PROPERTIES CATIONIC CELLULOSIC POLYMERS IN SHAMPOO SYSTEMS" JOURNAL OF COSMETIC SCIENCE, SOCIETY OF COSMETIC CHEMISTS, NEW YORK, NY,, US, vol. 55, no. SUPPL, 2004, pages 195-205, XP009076338 ISSN: 1525-7886

## Description

La présente invention a pour objet une composition de teinture d'oxydation des fibres kératiniques, et en particulier des fibres kératiniques humaines telles que les cheveux, comprenant un ou plusieurs éther(s) de cellulose cationique(s), un ou plusieurs métasilicate(s) et un ou plusieurs colorant(s) d'oxydation benzénique(s), hétérocyclique(s) ou naphtalénique(s).

L'invention a aussi pour objet l'utilisation de cette composition pour la teinture des fibres kératiniques ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho-ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (i.e. abîmées) de sa pointe à sa racine.

L'utilisation des éthers de cellulose cationiques dans des compositions de coloration d'oxydation pour fibres kératiniques est connue notamment par la demande de brevet WO 2006/099163.

Le but de la présente invention est l'obtention de compositions de coloration capillaire stables, notamment sous forme de crèmes, faciles à préparer et à appliquer, ayant de bonnes qualités rhéologiques et conduisant à des colorations peu sélectives et résistantes aux diverses agressions que peuvent subir les fibres kératiniques.

De manière surprenante et avantageuse, la Demanderesse a découvert que l'utilisation, en combinaison, d'un ou plusieurs éther(s) de cellulose cationique(s) particulier(s), d'un ou plusieurs métasilicate(s) et d'un ou plusieurs colorant(s) choisi(s) parmi les colorants d'oxydation benzéniques, hétérocycliques ou naphtaléniques permet d'obtenir des compositions de coloration capillaire de très bonne qualité aux propriétés améliorées.

Les compositions tinctoriales selon l'invention présentent notamment les propriétés suivantes :
- ces compositions tinctoriales peuvent comprendre des colorants sous forme de sels en concentrations élevées sans présenter de problèmes de stabilité,
- il est possible d'obtenir des compositions de viscosité correspondant à une crème qui sont stables dans le temps,
- ces compositions se distinguent par une facilité de mélange avec la composition oxydante,
- ces compositions se distinguent par les qualités rhéologiques des crèmes obtenues (bonne viscosité de crème en mélange),
- facilité d'application des compositions après mélange avec la composition oxydante au moment de la mise en oeuvre de la coloration (qualités d'usage sur tête).

En outre, les compositions selon l'invention permettent l'obtention de compositions capables de conduire à des colorations aux nuances variées, chromatiques, puissantes, esthétiques, peu sélectives, uniformes sur l'ensemble de la chevelure et résistant bien aux diverses agressions que peuvent subir les fibres.

Ces compositions sont aussi non-agressives pour le cuir chevelu lors de l'application.

La présente invention a pour objet une composition tinctoriale pour fibres kératiniques, et en particulier pour fibres kératiniques humaines telles que les cheveux, comprenant, dans un milieu approprié pour la teinture, un ou plusieurs éther(s) de cellulose cationique(s) particulier(s) décrit(s) ci-dessous, un ou plusieur(s) métasilicate(s), et un ou plusieurs colorant(s) d'oxydation choisi(s) parmi les colorants d'oxydation benzéniques, hétérocycliques et naphtaléniques.

Un autre objet de la présente invention consiste en un procédé de teinture des fibres kératiniques dans lequel la composition cosmétique selon l'invention est mise en oeuvre.

Un troisième objet de l'invention concerne l'utilisation de cette composition cosmétique pour la teinture des fibres kératiniques, et en particulier des fibres kératiniques humaines, telles que les cheveux.

D'autres caractéristiques, aspects, objets et avantages de la présente invention apparaîtront encore plus clairement à la lecture de la description et des exemples qui suivent.

La composition tinctoriale pour fibres kératiniques, selon l'invention, comprend, dans un milieu approprié pour la teinture :
A) un ou plusieurs éther(s) de cellulose cationique(s) comprenant de 4 000 à 10 000 motifs anhydroglucoses, lesdits motifs anhydroglucoses étant substitués par au moins :
   (i) un substituant de formule [R₄R₅R₆R₉N⁺](X₂⁻), dans laquelle :
      R₄ et R₅ représentent, indépendamment l'un de l'autre, un groupe méthyle ou éthyle,
      R₆ représente un groupe alkyle, linéaire ou ramifié, en C₈-C₂₄ ou aralkyle dont la partie alkyle, linéaire ou ramifiée, est en C₈-C₂₄,
      R₉ représente un groupe divalent permettant le rattachement au groupement anhydroglucose et choisi parmi -(B)q-CH₂-CHOH-CH₂- et -CH₂CH₂-,
      q désignant 0 ou 1,
      B désignant un groupe divalent -(CH₂CH₂O)_{n'}-,
      n' un nombre entier allant de 1 à 100,
      X₂⁻ représente un anion ; et
   (ii) un substituant de formule [R₁R₂R₃R₈N⁺](X₁⁻), dans laquelle :
      R₁, R₂, R₃ représentent, indépendamment l'un de l'autre, un groupe méthyle ou éthyle,
      R₈ représente un groupe divalent permettant le rattachement au groupement anhydroglucose et choisi parmi -(A)ₚ-CH₂-CHOH-CH₂- et -CH₂CH₂-,
      p désignant 0 ou 1,
      A désignant un groupe divalent -(CH₂CH₂O)ₙ-,
      n un nombre entier allant de 1 à 100,
      X₁⁻ représente un anion ;
B) un ou plusieurs métasilicate(s) ; et
C) un ou plusieurs colorant(s) d'oxydation choisi(s) parmi les colorants d'oxydation benzéniques, hétérocycliques et naphtaléniques.

De préférence, le substituant (i) de formule [R₄R₅R₆R₉N⁺](X₂⁻) est présent en une moyenne de 0,0003 à 0,08 mole, par mole d'unité anhydroglucose.

Les éthers de cellulose cationiques utilisables dans les compositions selon l'invention sont, de préférence, des hydroxyéthyl ou hydroxypropyl celluloses.

Les éthers de cellulose cationiques utilisables dans les compositions selon l'invention comprennent, de préférence, plus de 4 500, avantageusement, plus de 5000, et de manière plus préférée, plus de 6000 motifs anhydroglucoses.

De préférence, les éthers de cellulose cationiques utilisables dans les compositions selon l'invention comprennent, de préférence, jusqu'à 9000, et de manière préférée, jusqu'à 8000 motifs anhydroglucoses.

Ces éthers de cellulose cationiques et leur procédé de préparation sont décrits dans la demande WO 2005/000903.

Selon une variante préférée, les éthers de cellulose cationiques utilisables dans les compositions selon l'invention sont formés d'au moins un motif (IV) et d'au moins un des motifs (I), (II) ou (III) suivants : 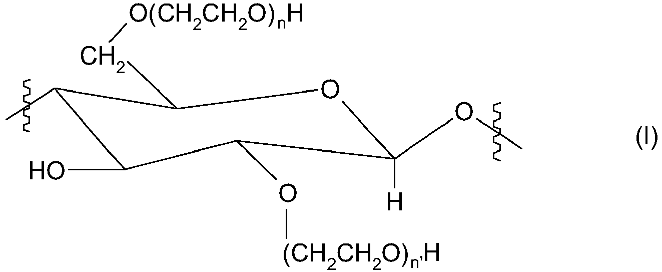 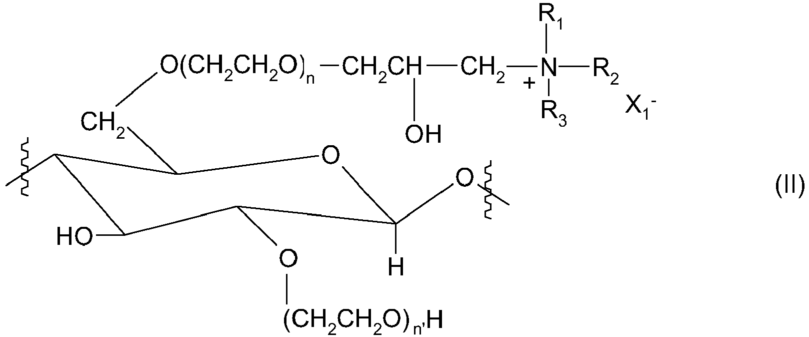 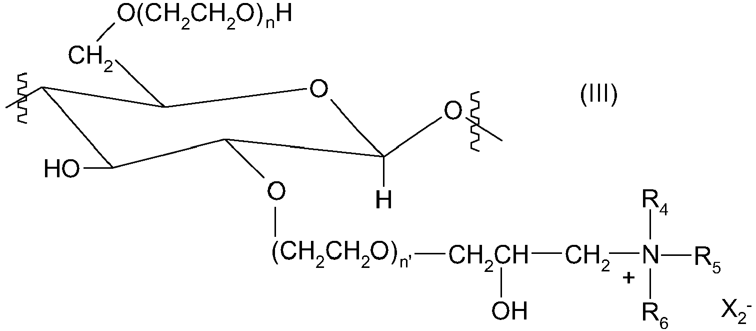 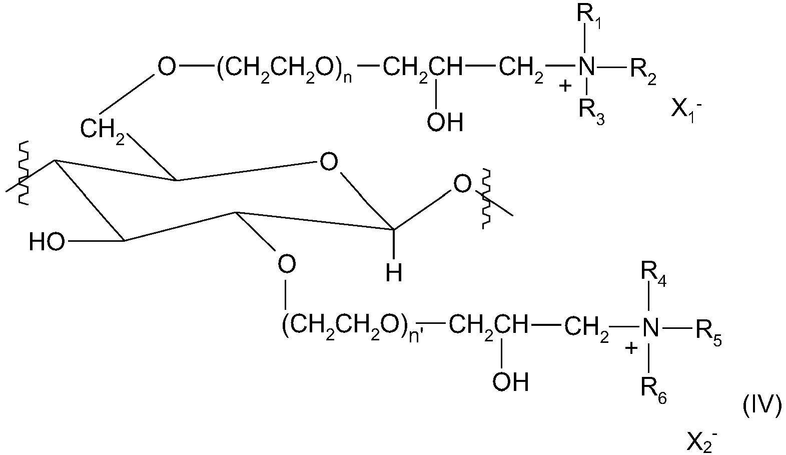 sous réserve que :
le nombre total des motifs (I) + (II) + (III) + (IV) soit compris entre 4000 et 10000 ;
le rapport [(III) + (IV)] / [(I) + (II) + (III) + (IV)] va de 0,0003 à 0,8 ;
le rapport [(II) + (IV)] / [(I) + (II) + (III) + (IV)] va de 0,02 à 0,9 ;
les nombres entiers n et n', indépendamment l'un de l'autre, vont de 0 à 5 ;
R₁, R₂, R₃, R₄ et R₅ représentent, indépendamment l'un de l'autre, un groupe méthyle ou éthyle ;
R₆ représente un groupement alkyle, linéaire ou ramifié, en C₈-C₂₄, de préférence en C₁₀-C₂₄, de manière plus préférée en C₁₂-C₂₄, et mieux encore en C₁₂-C₁₅, ou un groupement aralkyle dont la partie alkyle, linéaire ou ramifiée, est en C₈-C₂₄ ;
X₁⁻ et X₂⁻ représentent des anions choisis, de préférence, indépendamment l'un de l'autre, parmi les ions phosphate, nitrate, sulfate et halogénure (Cl⁻, Br⁻, F⁻, I⁻).

Selon une variante particulière, les éthers de cellulose cationiques utilisables dans les compositions selon l'invention, sont formés d'au moins un motif (IV) et d'au moins un des motifs (I), (II), ou (III) ci-dessus dans le(s)quel(s) R₆ est un groupement dodécyle linéaire.

Parmi les éthers de cellulose cationiques utilisables dans les compositions de l'invention, on peut citer les polymères de type SOFTCAT SL-5, SL-30, SL-60 et SL-100 (INCI : Polyquaternium-67) commercialisés par la société Amerchol. Les éthers de cellulose cationiques particulièrement préférés sont les polymères de type SL-60 et SL-100.

La composition selon l'invention peut comprendre un ou plusieurs éther(s) de cellulose cationique(s) tel(s) que défini(s) précédemment.

La concentration en éther(s) de cellulose cationique(s) des compositions selon l'invention va de préférence de 0,01 à 10 % en poids, en particulier de 0,05 à 3 % en poids, et de manière plus préférée de 0,1 à 1 % en poids, par rapport au poids total de la composition.

Le (ou les) métasilicate(s) utilisable(s) dans les compositions de l'invention répond(ent), de préférence, à la formule générale suivante :

(Y^{p+})ₙSiO₃²⁻

dans laquelle :
Y désigne un métal mono- ou divalent, de préférence un métal alcalin tel que par exemple Li, Na, K, ou alcalinoterreux tel que par exemple Ba, Mg, Ca, ou un groupe NH₄ ;
n = 1 ou 2, p = 1 ou 2, et en particulier, quand n = 1 alors p = 2 et quand n = 2 alors p = 1.

De manière avantageuse, le métasilicate selon l'invention est du métasilicate de sodium (Na⁺)₂SiO₃²⁻.

La concentration en métasilicate(s) des compositions selon l'invention va de préférence de 0,005 à 20 % en poids, en particulier de 0,1 à 10 % en poids, et de manière plus préférée de 0,2 à 5 % en poids, par rapport au poids total de la composition.

Le (ou les) colorant(s) d'oxydation utilisable(s) selon l'invention est (ou sont) choisi(s) parmi les colorants d'oxydation benzéniques, hétérocycliques et naphtaléniques.

Le (ou les) colorant(s) d'oxydation utilisable(s) dans les compositions de l'invention peut (ou peuvent) être notamment choisi(s) parmi les bases benzéniques cationiques ou non, les bases hétérocycliques, les coupleurs benzéniques, les coupleurs hétérocycliques et les coupleurs naphtaléniques.

De préférence, les compositions selon l'invention contiennent une ou plusieurs base(s) d'oxydation.

Les bases d'oxydation benzéniques peuvent être cationiques ou non-cationiques.

A titre de bases d'oxydation benzéniques non cationiques utilisables, on peut citer les para-phénylènediamines, les bis-phénylalkylènediamines, les para-aminophénols, les ortho-aminophénols, et leurs sels d'addition.

Parmi les para-phénylènediamines de ce type, on peut citer à titre d'exemple, la para-phénylènediamine, la para-toluylènediamine, la 2-chloro-para-phénylènediamine, la 2,3-diméthyl-para-phénylènediamine, la 2,6-diméthyl-para-phénylènediamine, la 2,6-diéthyl-para-phénylènediamine, la 2,5-diméthyl-para-phénylènediamine, la N,N-diméthyl-para-phénylènediamine, la N,N-diéthyl-para-phénylènediamine, la N,N-dipropyl-para-phénylènediamine, la 4-amino-N,N-diéthyl-3-méthyl-aniline, la N,N-bis-(β-hydroxyéthyl)-para-phénylènediamine, la 4-N,N-bis-(β-hydroxyéthyl)amino-2-méthylaniline, la 4-N,N-bis-(β-hydroxyéthyl)amino-2-chloroaniline, la 2-β-hydroxyéthyl-para-phénylènediamine, la 2-fluoro-para-phénylènediamine, la 2-isopropyl-para-phénylènediamine, la N-(β-hydroxypropyl)-para-phénylènediamine, la 2-hydroxyméthyl-para-phénylènediamine, la N,N-diméthyl-3-méthyl-para-phénylènediamine, la N,N-(éthyl, β-hydroxyéthyl)-para-phénylènediamine, la N-(β,γ-dihydroxypropyl)-para-phénylènediamine, la N-(4'-aminophényl)-para-phénylènediamine, la N-phényl-para-phénylènediamine, la 2-β-hydroxyéthyloxy-para-phénylènediamine, la 2-β-acétylaminoéthyloxy-para-phénylènediamine, la N-(β-méthoxyéthyl)-para-phénylènediamine, la 4-aminophénylpyrrolidine, la 2-thiényl para-phénylènediamine, le 2-β-hydroxyéthylamino-5-aminotoluène, la 3-hydroxy-1-(4'-aminophényl)pyrrolidine, et leurs sels d'addition.

Parmi les para-phénylènediamines citées ci-dessus, la para-phénylènediamine, la para-toluylènediamine, la 2-isopropyl-para-phénylènediamine, la 2-β-hydroxyéthyl-para-phénylènediamine, la 2-β-hydroxyéthyloxy-para-phénylènediamine, la 2,6-diméthyl-para-phénylènediamine, la 2,6-diéthyl-para-phénylènediamine, la 2,3-diméthyl-para-phénylènediamine, la N,N-bis-(β-hydroxyéthyl)-para-phénylènediamine, la 2-chloro para-phénylènediamine, la 2-β-acétylaminoéthyloxy-para-phénylènediamine, et leurs sels d'addition, sont particulièrement préférés.

Parmi les bis-phénylalkylènediamines non cationiques, on peut citer à titre d'exemple, le N,N'-bis-(β-hydroxyéthyl)-N,N'-bis-(4'-aminophényl) 1,3-diaminopropanol, la N,N'-bis-(β-hydroxyéthyl)-N,N'-bis-(4'-aminophényl)éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino, 3'-méthylphényl)éthylènediamine, le 1,8-bis-(2,5-diamino-phénoxy)-3,6-dioxaoctane, et leurs sels d'addition.

Parmi les para-aminophénols non-cationiques, on peut citer à titre d'exemple, le para-aminophénol, le 4-amino-3-méthylphénol, le 4-amino-3-fluorophénol, le 4-amino-3-hydroxyméthylphénol, le 4-amino-2-méthylphénol, le 4-amino-2-hydroxyméthylphénol, le 4-amino-2-méthoxyméthylphénol, le 4-amino-2-aminométhylphénol, le 4-amino-2-(β-hydroxyéthylaminométhyl)phénol, le 4-amino-2-fluorophénol, et leurs sels d'addition.

Parmi les ortho-aminophénols non-cationiques, on peut citer à titre d'exemple, le 2-aminophénol, le 2-amino-5-méthylphénol, le 2-amino-6-méthylphénol, le 5-acétamido-2-aminophénol, et leurs sels d'addition.

A titre de bases d'oxydation benzéniques cationiques utilisables dans les compositions selon l'invention, on peut citer les para-phénylènediamines telles que notamment décrites dans les demandes de brevets FR-A-2 766 177 et FR-A-2 766 178, les para-aminophénols tels que décrits par exemple dans les demandes de brevet FR-A-2 766 177 et FR-A-2 766 178, les ortho-phénylènediamines telles que décrites par exemple dans les demandes de brevet FR-A-2 782 718, FR-A-2 782 716 et FR-A-2 782 719, des ortho-aminophénols ou des bases doubles cationiques telles que des dérivés de type bis(aminophényl)alkylènediamine décrites dans les demandes de brevet FR-A-2 766 179, portant au moins un atome d'azote quaternaire.

De préférence, les bases d'oxydation benzéniques cationiques utilisables dans les compositions selon l'invention sont des para-phénylènediamines cationiques.

De manière avantageuse, une variante consiste à mettre en oeuvre des bases d'oxydation cationiques de structure para-phénylènediamine, dont au moins une des fonctions amine est une amine tertiaire, porteur d'un noyau pyrrolidinique, la molécule possédant au moins un atome d'azote quaternisé. De telles bases sont, par exemple, décrites dans le document EP-A-1 348 695.

Selon une variante, la composition tinctoriale selon l'invention comprend au moins une para-phénylènediamine cationique choisie parmi les composés suivants :
- [1-(4-aminophényl)pyrrolidin-3-yl]triméthylammonium ; chlorure,
- [1-(4-aminophényl)pyrrolidin-3-yl]diméthyltétradécylammonium ; bromure
- 3-[1-(4-aminophényl)pyrrolidin-3-yl]-1-méthyl-3H-imidazol-1-ium ; chlorure
- [1-(4-aminophényl)pyrrolidin-3-yl]-(2-hydroxyéthyl)diméthylammonium ; chlorure
- [1-(4-aminophényl)pyrrolidin-3-yl]diméthyl-(3-triméthylsilanylpropyl)ammonium ; chlorure
- [1-(4-aminophényl)pyrrolidin-3-yl]-(3-triméthylammonium-hexyl)diméthylammonium ; dichlorure
- {2-[1-(4-aminophényl)pyrrolidin-3-yloxy]éthyl}triméthylammonium ; chlorure
- 1-{2-[1-(4-aminophényl)pyrrolidin-3-yloxy]éthyl}-1-méthyl-pyrrolidinium ; chlorure
- 3-{3-[1-(4-aminophényl)pyrrolidin-3-yloxy]propyl}-1-méthyl-3H-imidazol-1-ium ; chlorure
- 1-{2-[1-(4-aminophényl)pyrrolidin-3-yloxy]éthyl}-1-méthyl-pipéridinium ; chlorure
- 3-{3-[1-(5-triméthylsilanyléthyl-4-amino-3-triméthylsilanyléthylphényl)pyrrolidin-3-yloxy]propyl}-1-méthyl-3H-imidazol-1-ium ; chlorure
- [1-(4-amino-3-méthylphényl)pyrrolidin-3-yl]triméthylammonium ; chlorure
- [1-(4-amino-3-méthylphényl)pyrrolidin-3-yl]diméthyltétradécylammonium ; chlorure
- 3-[1-(4-amino-3-méthylphényl)pyrrolidin-3-yl]-1-méthyl-3H-imidazol-1-ium ; chlorure
- [1-(4-amino-3-méthylphényl)pyrrolidin-3-yl]-(2-hydroxyéthyl)-diméthylammonium ; chlorure
- [1-(4-amino-3-méthylphényl)pyrrolidin-3-yl]-diméthyl-(3-triméthylsilanylpropylammonium ; chlorure
- [1-(4-amino-3-méthylphényl)pyrrolidin-3-yl]-(3-triméthylammoniumhexyl)diméthylammonium ; dichlorure
- {2-[1-(4-amino-3-méthylphényl)pyrrolidin-3-yloxy]éthyl}-triméthylammonium ; chlorure
- 1-{2-[1-(4-amino-3-méthylphényl)pyrrolidin-3-yloxy]éthyl}-1-méthylpyrrolidinium ; chlorure
- 3-{3-[1-(4-amino-3-méthylphényl)pyrrolidin-3-yloxy]propyl}-1-méthyl-3H-imidazol-1-um ; chlorure
- 1-{2-[1-(4-amino-3-méthylphényl)pyrrolidin-3-yloxy]éthyl}-1-méthylpipéridinium ; chlorure
- [1-(4-amino-3-triméthylsilanyléthylphényl)pyrrolidin-3-yl]-triméthylammonium ; chlorure
- 3-[1-(4-amino-3-triméthylsilanyléthylphényl)pyrrolidin-3-yl]-1-méthyl-3H-imidazol-1-ium ; chlorure
- 3-{3-[1-(4-amino-3-triméthylsilanyléthylphényl)pyrrolidin-3-yl-oxy]propyl}-1-méthyl-3H-imidazol-1-um ; chlorure
- [1-(5-triméthylsilanyléthyl-4-amino-3-triméthylsilanyléthylphényl)pyrrolidin-3-yl]triméthylammonium ; chlorure
- 3-[1-(5-triméthylsilanyléthyl-4-amino-3-triméthylsilanyléthylphényl)pyrrolidin-3-yl]-1-méthyl-3H-imidazol-1-ium ; chlorure
- 1'-(4-aminophényl)-1-méthyl-[1,3']-bipyrrolidinyl-1-ium ; chlorure
- 1'-(4-amino-3-méthylphényl)-1-méthyl-[1,3']-bipyrrolidinyl-1-ium ; chlorure
- 3-{[1-(4-aminophényl)pyrrolidin-3-ylcarbamoyl]méthyl}-1-méthyl-3H-imidazol-1-ium ; chlorure
- 3-{[1-(4-amino-3-méthylphényl)pyrrolidin-3-ylcarbamoyl]-méthyl}-1-méthyl-3H-imidazol-1-ium ; chlorure
- 3-[1-(4-aminophényl)pyrrolidin-3-yl]-1-(3-triméthylsilanylpropyl)-3H-imidazol-1-ium ; chlorure
- 3-[1-(4-aminophényl)pyrrolidin-3-yl]-1-(3-triméthylsilanylpropyl)-3H-imidazol-1-ium ; chlorure
- [1-(4-aminophényl)pyrrolidin-3-yl]éthyldiméthylammonium ; chlorure
- [1-(4-aminophényl)pyrrolidin-3-yl]éthyldiméthylammonium ; iodure
- [1-(4-aminophényl)pyrrolidin-3-yl]propyldiméthylammonium ; iodure
- [1-(4-aminophényl)pyrrolidin-3-yl]propyldiméthylammonium ; bromure
- [1-(4-aminophényl)pyrrolidin-3-yl]propyldiméthylammonium ; méthosulfate
- [1-(4-aminophényl)pyrrolidin-3-yl]-butyldiméthylammonium ; iodure
- [1-(4-aminophényl)pyrrolidin-3-yl]pentyldiméthylammonium ; iodure
- [1-(4-aminophényl)pyrrolidin-3-yl]hexyldiméthylammonium ; iodure
- [1-(4-aminophényl)pyrrolidin-3-yl]heptyldiméthylammonium ; iodure
- [1-(4-aminophényl)pyrrolidin-3-yl]octyldiméthylammonium ; iodure
- [1-(4-aminophényl)pyrrolidin-3-yl]décyldiméthylammonium ; iodure
- [1-(4-aminophényl)pyrrolidin-3-yl]hexadécyldiméthylammonium ; iodure
- [1-(4-aminophényl)pyrrolidin-3-yl]hydroxyéthyldiméthylammonium ; chlorure
- [1-(4-aminophényl)pyrrolidin-3-yl]hydroxyéthyldiméthylammonium ; iodure.

Par "base hétérocyclique" ou "base d'oxydation hétérocyclique", on entend, au sens de la présente invention, toute base d'oxydation comportant au moins un groupe hétérocyclique, différent d'un groupe pyrrolidinyle dont l'atome d'azote est substitué par un groupement para-aminophényle et possédant au moins un atome d'azote quaternisé.

A titre de bases d'oxydation hétérocycliques utilisables dans les compositions selon l'invention, on peut citer les pyridines, les pyrimidines, les pyrazoles, les pyrazolopyrimidines condensées, les pyrazolotriazoles, les pyrazolotétrazoles, les pyrazolopyridazines, les pyrazolothiazoles, les pyrazoloimidazoles, les pyrazolobenzimidazoles, les pyrazoloquinolines, les aminopyrolidines, les aminopyrazolines, les mono- ou diaminotétraquinoléines, les diamino- ou triaminoquinoléines, les aminoindazoles, les diaminouraciles, les aminoindolénines, les hydrazones, la julolidine ou la lilolidine, ainsi que leurs dérivés et leurs sels d'addition.

Parmi les dérivés pyridiniques, on peut citer les composés décrits par exemple dans les brevets GB-A-1 026 978 et GB-A-1 153 196, comme la 2,5-diaminopyridine, la 2-(4-méthoxyphényl)amino-3-aminopyridine, la 2,3-diamino-6-méthoxypyridine, la 2-(β-méthoxyéthyl)amino-3-amino-6-méthoxypyridine, la 3,4-diaminopyridine, et leurs sels d'addition.

D'autres bases d'oxydation pyridiniques utilisables dans la présente invention sont les 3-aminopyrazolo[1,5-a]pyridines ou leurs sels d'addition, décrits, par exemple, dans la demande de brevet FR-A-2 801 308. A titre d'exemple, on peut citer la pyrazolo[1,5-a]-pyridin-3-ylamine ; la 2-acétylaminopyrazolo[1,5-a]pyridin-3-ylamine ; la 2-morpholin-4-yl-pyrazolo[1,5-a]pyridin-3-ylamine ; l'acide 3-aminopyrazolo[1,5-a]pyridin-2-carboxylique ; la 2-méthoxypyrazolo-[1,5-a]pyridine-3-ylamino ; le (3-aminopyrazolo[1,5-a]pyridine-7-yl)méthanol ; le 2-(3-aminopyrazolo[1,5-a]pyridine-5-yl)éthanol ; le 2-(3-aminopyrazolo-[1,5-a]-pyridine-7-yl)éthanol ; le (3-amino-pyrazolo[1,5-a]pyridine-2-yl)méthanol ; la 3,6-diaminopyrazolo-[1,5-a]pyridine ; la 3,4-diaminopyrazolo[1,5-a]pyridine ; la pyrazolo-[1,5-a]pyridine-3,7-diamine ; la 7-morpholin-4-yl-pyrazolo[1,5-a]-pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)amino]éthanol ; le 2-[(3-aminopyrazolo[1,5-a]pyridin-7-yl)-(2-hydroxyéthyl)amino]éthanol ; la 3-aminopyrazolo[1,5-a]pyridine-5-ol ; 3-aminopyrazolo[1,5-a]pyridine-4-ol ; la 3-aminopyrazolo[1,5-a]pyridine-6-ol ; la 3-aminopyrazolo[1,5-a]pyridine-7-ol, ainsi que leurs sels d'addition.

Parmi les dérivés pyrimidiniques, on peut citer les composés décrits par exemple dans les brevets DE-A-2359399 ; JP 88-169571 ; JP 05-63124 ; EP-A-0770375 ou demande de brevet WO 96/15765 comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolopyrimidiniques tels ceux mentionnés dans la demande de brevet FR-A-2 750 048 et parmi lesquels on peut citer la pyrazolo-[1,5-a]pyrimidine-3,7-diamine ; la 2,5-diméthylpyrazolo[1,5-a]-pyrimidine-3,7-diamine ; la pyrazolo[1,5-a]pyrimidine-3,5-diamine ; la 2,7-diméthylpyrazolo[1,5-a]pyrimidine-3,5-diamine ; le 3-amino-pyrazolo[1,5-a]pyrimidin-7-ol ; le 3-aminopyrazolo[1,5-a]-pyrimidin-5-ol ; le 2-(3-aminopyrazolo[1,5-a]pyrimidin-7-ylamino)-éthanol, le 2-(7-aminopyrazolo[1,5-a]pyrimidin-3-ylamino)éthanol, le 2-[(3-aminopyrazolo[1,5-a]pyrimidin-7-yl)(2-hydroxyéthyl)amino]-éthanol, le 2-[(7-aminopyrazolo[1,5-a]pyrimidin-3-yl)(2-hydroxyéthyl)amino]éthanol, la 5,6-diméthylpyrazolo[1,5-a]pyrimidine-3,7-diamine, la 2,6-diméthylpyrazolo[1,5-a]pyrimidine-3,7-diamine, la 2, 5, N7, N7-tétraméthylpyrazolo[1,5-a]pyrimidine-3,7-diamine, la 3-amino-5-méthyl-7-imidazolylpropylaminopyrazolo[1,5-a]pyrimidine, leurs sels d'addition, et leurs formes tautomères lorsqu'il existe un équilibre tautomérique.

Parmi les dérivés pyrazoliques, on peut citer les composés décrits dans les brevets DE-A-38 43 892, DE-A-41 33 957 et demandes de brevet WO 94/08969, WO 94/08970, FR-A-2 733 749 et DE-A-195 43 988 comme le 4,5-diamino-1-méthylpyrazole, le 4,5-diamino-1-(β-hydroxyéthyl)pyrazole, le 3,4-diaminopyrazole, le 4,5-diamino-1-(4'-chlorobenzyl)pyrazole, le 4,5-diamino-1,3-diméthyl-pyrazole, le 4,5-diamino-3-méthyl-1-phénylpyrazole, le 4,5-diamino-1-méthyl-3-phénylpyrazole, le 4-amino-1,3-diméthyl-5-hydrazino-pyrazole, le 1-benzyl-4,5-diamino-3-méthylpyrazole, le 4,5-diamino-3-tert-butyl-1-méthylpyrazole, le 4,5-diamino-1-tert-butyl-3-méthyl-pyrazole, le 4,5-diamino-1-(β-hydroxyéthyl)-3-méthylpyrazole, le 4,5-diamino-1-éthyl-3-méthylpyrazole, le 4,5-diamino-1-éthyl-3-(4'-méthoxyphényl)pyrazole, le 4,5-diamino-1-éthyl-3-hydroxy-méthylpyrazole, le 4,5-diamino-3-hydroxyméthyl-1-méthylpyrazole, le 4,5-diamino-3-hydroxyméthyl-1-isopropylpyrazole, le 4,5-diamino-3-méthyl-1-isopropylpyrazole, le 4-amino-5-(2'-aminoéthyl)amino-1,3-diméthylpyrazole, le 3,4,5-triaminopyrazole, le 1-méthyl-3,4,5-triaminopyrazole, le 3,5-diamino-1-méthyl-4-méthylaminopyrazole, le 3,5-diamino-4-(β-hydroxyéthyl)amino-1-méthylpyrazole, et leurs sels d'addition.

A titre de pyrazolotriazoles, on peut citer les composés 3-amino-4-méthyl-6-méthylthio-2-phénylpyrazolo[3,2-c]-s-triazole, 3-amino-2,4,6-triméthylpyrazolo[3,2-c]-s-triazole, 3-amino-4,6-diméthyl-pyrazolo[3,2-c]-s-triazole. De tels composés sont décrits dans le document US 5,457,200. On peut aussi citer les composés 7-amino-2,6-diméthylpyrazolo[1,5-b]-1,2,4-triazole, 7-amino-3,6-diméthylpyrazolo-[3,2-c]-1,2,4-triazole, 7-amino-3-méthylpyrazolo[3,2-c]-1,2,4-triazole, 7-amino-3-méthyl-6-carboxypyrazolo[3,2-c]-1,2,4-triazole, 7-amino-2-méthylpyrazolo[1,5-b]-1,2,4-triazole, 7-amino-2-phénylpyrazolo-[1,5-b]-1,2,4-triazole, 7-amino-2-méthyl-6-carboxypyrazolo[1,5-b]-1,2,4-triazole. Ces composés sont décrits dans la demande de brevet EP-A-923 929.

A titre de pyrazolotétrazoles, on peut citer les composés 7-amino-6-méthylpyrazolo[1,5-e]tétrazole, 7-amino-6-phénylpyrazolo-[1,5-e]tétrazole, 7-amino-6-carboxypyrazolo[1,5-e]tétrazole décrits dans la demande de brevet EP-A-923 929.

A titre de pyrazolopyridazine, on peut citer la 3-aminopyrazolo-[1,5-b]pyridazine. De tels composés sont décrits dans le document US 5,457,200.

A titre de pyrazolothiazoles, on peut citer les composés 3-amino-2-méthylpyrazolo[3,2-b]thiazole, 3-aminopyrazolo[3,2-b]-thiazole, 3-amino-2,5-diméthyl-6-phénylpyrazolo[3,2-b]thiazole. De tels composés sont décrits dans le document US 5,427,200.

A titre de pyrazoloimidazoles, on peut citer les composés 3-amino-4-benzylpyrazolo[1,5-a]imidazole, 3-amino-2,4-diméthyl-pyrazolo[1,5-a]imidazole, 3-amino-4-méthylpyrazolo[1,5-a]imidazole. De tels composés sont décrits dans le document US 5,457,200. On peut aussi citer les composés 7-amino-6-méthylpyrazolo[1,5-a]imidazole, 7-aminopyrazolo[1,5-a]imidazole, 7-amino-2-méthylpyrazolo[1,5-a]-imidazole, 7-amino-2-phénylpyrazolo[1,5-a]imidazole décrits dans la demande de brevet EP-A-923 929.

A titre de pyrazolobenzimidazoles, on peut citer les composés 7-amino-6-méthylpyrazolo[1,5-a]benzimidazole, 6,7-diaminopyrazolo-[1,5-a]benzimidazole, 6,7-diamino-2-méthylpyrazolo[1,5-a]-benzimidazole, 6,7-diamino-2-phénylpyrazolo[1,5-a]benzimidazole décrits, par exemple, dans la demande de brevet EP-A-923 929.

A titre de pyrazoloquinolines, on peut citer les composés 3-amino-2-phénylpyrazolo[1,5-a]quinoline. De tels composés sont décrits dans le document US 5,457,200.

A titre d'aminopyrazolines, on peut citer les composés 1-(4'-aminophényl)-3-aminopyrazoline, 1-(4'-hydroxyphényl)-3-amino-pyrazoline. De tels composés sont décrits dans le document FR-A-2 018 056.

A titre de mono- ou diaminotétrahydroquinoléines, on peut citer les composés 5-amino-1,2,3,4-tétrahydroquinoléine, 5-amino-7-chloro-8-pipéridino-1,2,3,4-tétrahydroquinoléine, 5-amino-7-chloro-8-morpholino-1,2,3,4-tétrahydroquinoléine, 5,7-diamino-6-méthyl-8-hydroxy-1,2,3,4-tétrahydroquinoléine, 5-amino-8-méthoxy-1,2,3,4-tétrahydroquinoléine, 5-amino-7-chloro-8-diméthylamino-1,2,3,4-tétrahydroquinoléine. De tels composés sont décrits dans le document DE-A-24 41 895.

A titre de diaminoquinoléines, on peut citer les composés 5,7-diamino-6-méthyl-8-hydroxyquinoléine et 5,7-diamino-2-méthyl-8-hydroxyquinoléine. De tels composés sont décrits dans le document DE-A-24 41 598.

A titre de triaminoquinoléine, on peut citer 5,7-diamino-8-méthylaminoquinoléine, 5,7-diamino-8-diméthylaminoquinoléine, 5,7-diamino-8-morpholinoquinoléine, 5,7-diamino-8-béta-hydroxyéthylaminoquinoléine, 5,7,8-triaminoquinoléine. De tels composés sont décrits dans le document DE-A-2441599.

A titre d'aminoindazoles, on peut citer 4,7-diamino-5-méthylindazole, 4,7-diamino-5,6-diméthylindazole, 6,7-diaminoindazole, 6-hydroxy-7-aminoindazole, 1-éthyl-6-hydroxy-7-aminoindazole, 6-aminoindazole, 5,6-diaminoindazole. De tels composés sont décrits dans le document FR-A-2 315 906, DE-A-14 92 166.

A titre de diaminouraciles, on peut citer les composés 5,6-diaminouracile, 5,6-diamino-2-thiouracile, 5,6-diamino-3-méthyl-uracile, 5-amino-3-méthyl-6-méthylaminouracile, 5-amino-3-méthyl-6-béta-hydroxyéthylaminouracile, 5-amino-3-méthyl-6-benzylamino-uracile, 5-amino-3-méthyl-6-phénylaminouracile, 5,6-diamino-1-phényluracile, 5,6-diamino-1,3-diméthyluracile, 5-amino-1,3-diméthyl-6-méthylaminouracile, 5-amino-1,3-diméthyl-6-béta-hydroxyméthylaminouracile, 5-amino-1,3-diméthyl-6-benzylaminouracile, 5-amino-1,3-diméthyl-6-phénylaminouracile, 5-amino-1,3-diméthyl-6-diméthylaminouracile. De tels composés sont décrits dans le document DE-A-25 33 629.

A titre d'aminoindolénines, on peut citer les composés 2-méthyl-5-aminoindolénine, 1-béta-hydroxyéthyl-2-méthyl-5-amino-indolénine. De tels composés sont décrits dans le document FR-A-1 602 547.

A titre d'hydrazones, on peut citer les composés N-méthylpyridone-4-hydrazone, N-méthylthiazolonehydrazone, N-méthylthiazo lone-2-hydrazone, N,N-diméthylbenzimidazolone-hydrazone, N-méthylpyridone-2-hydrazone, N-méthylbenzothiazolone-2-hydrazone, 1,2-diméthylindazolone-3-hydrazone, 1,2,6-triméthyl-pyridone-4-hydrazone, 1-méthylquinolone-2-hydrazone, 1,2,6-triméthyl-3-nitropyridone-4-hydrazone, 1,2,6-triméthyl-3-amino-pyridone-4-hydrazone, N-méthylcyclohexénothiazolonehydrazone, 1,2,5-triméthylpyrazolone-3-hydrazone, 1,2-diméthylindazolone-3-hydrazone, 1,2-diméthyl-5-chloroindazolone-3-hydrazone, 1-méthyl-2-éthyl-5-nitroindazolone-3-hydrazone, N-méthylquinolone-4-hydrazone, N-méthylbenzothiazolone-2-oméga-benzènesulfonylhydrazone. De tels composés sont décrits dans le document FR-A-1 602 547.

A titre de dérivés de julolidine ou de lilolidine, on peut citer les composés 9-aminojulolidine, 9-amino-8-méthyljulolidine, 9-amino-8,10-diméthyljulolidine, 8-aminolilolidine. De tels composés sont décrits dans le document DE-A-24 41 597.

De préférence, les bases d'oxydation hétérocycliques utiles pour la présente invention sont choisies parmi les pyridines, les pyrimidines, les pyrazoles et les pyrazolopyrimidines.

Encore plus préférentiellement, elles sont choisies parmi les 4,5-diaminopyrazoles.

De manière particulièrement préférée, les bases d'oxydation benzéniques ou hétérocycliques utilisées dans les compositions selon l'invention sont choisies parmi les para-phénylènediamines cationiques ou non, les para-aminophénols cationiques ou non, les dérivés pyrazoliques, ainsi que leurs sels d'addition.

Encore plus préférentiellement, les bases d'oxydation utilisées dans les compositions selon l'invention sont choisies parmi les dérivés pyrazoliques et leurs sels d'addition.

A titre de coupleurs benzéniques utilisables dans les compositions selon l'invention, on peut citer les méta-aminophénols, les méta-phénylènediamines, les méta-diphénols, ainsi que leurs sels d'addition.

Les méta-aminophénols, pouvant être utilisés, à titre de coupleurs benzéniques, dans les compositions tinctoriales conformes à l'invention, sont, de préférence, choisis parmi les composés de formule (V) suivante : 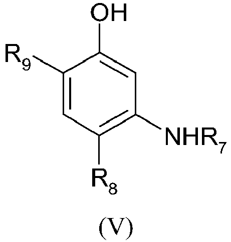 dans laquelle :
R₇ représente un atome d'hydrogène, un groupe alkyle en C₁-C₄, monohydroxyalkyle en C₁-C₄ ou polyhydroxyalkyle en C₂-C₄ ;
R₈ représente un atome d'hydrogène, un groupe alkyle en C₁-C₄, alcoxyle en C₁-C₄ ou un atome d'halogène choisi parmi le chlore, le brome ou le fluor ;
R₉ représente un atome d'hydrogène, un groupe alkyle en C₁-C₄, alcoxyle en C₁-C₄, monohydroxyalkyle en C₁-C₄, polyhydroxyalkyle en C₂-C₄, monohydroxyalcoxyle en C₁-C₄ ou polyhydroxyalcoxyle en C₂-C₄ ;
et parmi leurs sels d'addition.

Parmi les méta-aminophénols de formule (V) ci-dessus, on peut, plus particulièrement, citer le méta-aminophénol, le 5-amino-2-méthoxyphénol, le 5-amino-2-(β-hydroxyéthyloxy)phénol, le 5-amino-2-méthylphénol, le 5-N-(β-hydroxyéthyl)amino-2-méthylphénol, le 5-N-(β-hydroxyéthyl)amino-4-méthoxy-2-méthylphénol, le 5-amino-4-méthoxy-2-méthylphénol, le 5-amino-4-chloro-2-méthylphénol, le 5-amino-2,4-diméthoxyphénol, le 5-(y-hydroxypropylamino)-2-méthylphénol, et leurs sels d'addition.

Les méta-phénylènediamines, pouvant être utilisées à titre de coupleurs benzéniques dans la composition tinctoriale conforme à l'invention sont, de préférence, choisies parmi les composés de formule (VI) suivante : 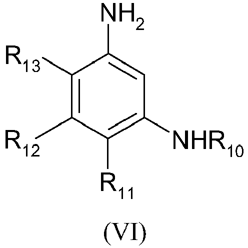 dans laquelle :
R₁₀ représente un atome d'hydrogène, un groupe alkyle en C₁-C₄, monohydroxyalkyle en C₁-C₄ ou polyhydroxyalkyle en C₂-C₄ ;
R₁₁ et R₁₂, identiques ou différents, représentent un atome d'hydrogène, un groupe alkyle en C₁-C₄, monohydroxyalcoxyle en C₁-C₄ ou polyhydroxyalcoxyle en C₂-C₄ ;
R₁₃ représente un atome d'hydrogène, un groupe alcoxyle en C₁-C₄, aminoalcoxyle en C₁-C₄, monohydroxyalcoxyle en C₁-C₄, polyhydroxyalcoxyle en C₂-C₄ ou un groupe 2,4-diaminophénoxyalcoxyle ;
et parmi leurs sels d'addition.

Parmi les méta-phénylènediamines de formule (VI) ci-dessus, on peut, plus particulièrement citer le 2,4-diaminobenzène, le 3,5-diamino-1-éthyl-2-méthoxybenzène, le 3,5-diamino-2-méthoxy-1-méthylbenzène, le 2,4-diamino-1-éthoxybenzène, le 1,3-bis-(2,4-diaminophénoxy)propane, le bis-(2,4-diaminophénoxy)-méthane, le 1-(β-aminoéthyloxy)-2,4-diaminobenzène, le 2-amino-1-(β-hydroxyéthyloxy)-4-méthylaminobenzène, le 2,4-diamino-1-éthoxy-5-méthylbenzène, le 2,4-diamino-5-(β-hydroxyéthyloxy)-1-méthyl-benzène, le 2,4-diamino-1-(β,γ-dihydroxypropyloxy)benzène, le 2,4-diamino-1-(β-hydroxyéthyloxy)benzène, le 2-amino-4-N-(β-hydroxyéthyl)amino-1-méthoxybenzène, et leurs sels d'addition.

Les méta-diphénols, pouvant être utilisés, à titre de coupleurs benzéniques dans les compositions tinctoriales conformes à l'invention, sont, de préférence, choisis parmi les composés de formule (VII) suivante : 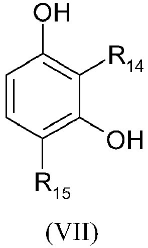 dans laquelle :
R₁₄ et R₁₅, identiques ou différents, représentent un atome d'hydrogène, un groupe alkyle en C₁-C₄ ou un atome d'halogène choisi parmi le chlore, le brome ou le fluor ;
et parmi leurs sels d'addition.

Parmi les méta-diphénols de formule (VII) ci-dessus, on peut, plus particulièrement, citer le 1,3-dihydroxybenzène, le 2-méthyl-1,3-dihydroxybenzène, le 4-chloro-1,3-dihydroxybenzène, le 2-chloro-1,3-dihydroxybenzène, et leurs sels d'addition.

A titre de coupleurs hétérocycliques utilisables dans les compositions selon l'invention, on peut citer les coupleurs hétérocycliques azolés, les coupleurs pyridiniques, les thiophènes, les indolines, les indoles, les benzofuranes, les 8-amino-6-méthoxyquinoléines, les 4-hydroxyquinolones, les benzodioxoles, les hydroxybenzamides, le sésamol et ses dérivés, les benzomorpholines, ainsi que leurs sels d'addition.

Les coupleurs hétérocycliques azolés utilisés dans les compositions selon l'invention peuvent être, en particulier, choisis parmi les carbazoles, les hydroxyindazoles, les benzoxazoles, les pyrazoloazoles et les pyrazolo triazoles, les pyrroloazoles, les imidazoloazoles, les thiazoloazoles, les pyrrolooxazoles, les hydroxypyrazolopyrimidines, les isoxazolones, les indazolones et les benzimidazoles.

A titre de carbazoles utilisés dans les compositions de l'invention, on peut citer le 1,3,6,8-tétraaminocarbazole, le 1,3,6,8-tétraamino-9-n-propylcarbazole, le 1,3,6,8-tétraamino-9-β-hydroxy-éthylcarbazole, le 1,3,6,8-tétraamino-9-(2'-N,N-diméthylaminoéthyl)-carbazole, et leurs sels d'addition. Ces composés sont décrits dans la demande DE-A-27 15 680.

On peut aussi citer, à titre de carbazoles, le 3-aminocarbazole décrit dans la demande DE-A-277 496.

A titre d'hydroxyindazoles utilisés, de manière préférée, dans les compositions selon l'invention, on peut citer les monohydroxyindazoles suivants : la 4-hydroxyindazole, la 5-hydroxyindazole, la 6-hydroxyindazole, la 7-hydroxyindazole, la 7-hydroxy-1-méthylindazole, la 4-hydroxy-6-méthylindazole, la 7-hydroxy-6-méthylindazole, la 7-hydroxy-4,6-diméthylindazole, la 6-hydroxy-7-bromoindazole, la 6-hydroxy-7-chloroindazole et la 6-hydroxy-5,7-dichloroindazole. Ces hydroxyindazoles sont décrits dans la demande de brevet DE-A-26 23 564.

A titre de benzoxazoles utilisés dans les compositions selon l'invention, on peut citer les diaminobenzoxazoles suivants : la 5,7-diaminobenzoxazole, la 5,7-diamino-2-méthylbenzoxazole, la 5,7-diamino-2-éthylbenzoxazole, la 5,7-diamino-2-butylbenzoxazole, la 5-diméthylamino-7-aminobenzoxazole, la 5-amino-7-diéthylamino-benzoxazole et la 4,6-diaminobenzoxazole. Ces benzoxazoles sont décrits dans la demande de brevet DE-A-27 19 424.

A titre de pyrazoloazoles utilisés dans les compositions selon l'invention, on peut citer les pyrazolo[1,5-b]-1,2,4-triazoles, les pyrazolo[3,2-c]-1,2,4-triazoles, les pyrazolotétrazoles, les pyrazolo[1,5-a]imidazoles, les pyrazolo[1,5-e]pyrazoles et les pyrazolo[1,5-e]-1,2,3-triazoles.

De manière préférée, les pyrazolo[1,5-b]-1,2,4-triazoles sont choisis parmi les 2-méthylpyrazolo[1,5-b]-1,2,4-triazole, 2-éthylpyrazolo[1,5-b]-1,2,4-triazole, 2-isopropylpyrazolo[1,5-b]-,2,4-triazole, 2-phénylpyrazolo[1,5-b]-,2,4-triazole, 2,6-diméthylpyrazolo-[1,5-b]-1,2,4-triazole, 6-méthyl-2-éthylpyrazolo-[1,5-b]-1,2,4-triazole, 6-méthyl-2-isopropylpyrazolo[1,5-b]-1,2,4-triazole, 6-méthyl-2-phénylpyrazolo[1,5-b]-1,2,4-triazole, 6-carboxy-2-méthylpyrazolo-[1,5-b]-1,2,4-triazole, 6-carboxy-2-éthylpyrazolo[1,5-b]-1,2,4-triazole, 6-carboxy-2-isopropylpyrazolo[1,5-b]-1,2,4-triazole, 6-carboxy-2-phénylpyrazolo[1,5-b]-1,2,4-triazole, 6-phényl-2-méthylpyrazolo-[1,5-b]-1,2,4-triazole, 6-phényl-2-éthylpyrazolo[1,5-b]-1,2,4-triazole, 6-phényl-2-isopropylpyrazolo[1,5-b]-1,2,4-triazole, 6-phényl-2-phényl-pyrazolo[1,5-b]-1,2,4-triazole, 6-amino-2-méthylpyrazolo[1,5-b]-1,2,4-triazole, 6-amino-2-éthylpyrazolo[1,5-b]-1,2,4-triazole, 6-amino-2-isopropylpyrazolo[1,5-b]-1,2,4-triazole, 6-amino-2-phénylpyrazolo-[1,5-b]-1,2,4-triazole, 6-éthylthio-2-méthylpyrazolo[1,5-b]-1,2,4-triazole, 6-éthylthio-2-éthylpyrazolo[1,5-b]-1,2,4-triazole, 6-éthylthio-2-isopropylpyrazolo[1,5-b]-1,2,4-triazole, 6-éthylthio-2-phényl-pyrazolo[1,5-b]-1,2,4-triazole, 6-éthoxy-2-méthylpyrazolo[1,5-b]-1,2,4-triazole, 6-éthoxy-2-éthylpyrazolo[1,5-b]-1,2,4-triazole, 6-éthoxy-2-isopropylpyrazolo[1,5-b]-1,2,4-triazole, 6-éthoxy-2-phénylpyrazolo[1,5-b]-1,2,4-triazole, 6-méthyl-2-(2'-aminoéthyl)-pyrazolo[1,5-b]-1,2,4-triazole, 6-carboxy-2-(2'-aminoéthyl)pyrazolo-[1,5-b]-1,2,4-triazole, 6-phényl-2-(2'-aminoéthyl)pyrazolo[1,5-b]-1,2,4-triazole, 6-éthylthio-2-(2'-aminoéthyl)pyrazolo[1,5-b]-1,2,4-triazole, 2-(2'-aminoéthyl)pyrazolo[1,5-b]-1,2,4-triazole, 2-(2'-hydroxyéthyl)pyrazolo[1,5-b]-1,2,4-triazole, 6-méthyl-2-(2'-hydroxyéthyl)pyrazolo[1,5-b]-1,2,4-triazole, 6-éthylthio-2-(2'-hydroxyéthyl)-pyrazolo[1,5-b]-1,2,4-triazole, 6-carboxy-2-(2'-hydroxyéthyl)pyrazolo-[1,5-b]-1,2,4-triazole, 6-phényl-2-(2'-hydroxyéthyl)pyrazolo[1,5-b]-1,2,4-triazole, 7-chloro-2,6-diméthylpyrazolo[1,5-b]-1,2,4-triazole, 7-bromo-2,6-diméthylpyrazolo[1,5-b]-1,2,4-triazole, et leurs sels d'addition.

De manière préférée, les pyrazolo[3,2-c]-1,2,4-triazoles sont choisis parmi les 3-méthylpyrazolo[3,2-c]-1,2,4-triazole, 3-méthylsulfinyl-6-phénylpyrazolo[3,2-c]-1,2,4-triazole, 3-éthyl-pyrazolo[3,2-c]-1,2,4-triazole, 3-isopropylpyrazolo[3,2-c]-1,2,4-triazole, 3-phénylpyrazolo[3,2-c]-1,2,4-triazole, 3-(2'aminoéthyl)-pyrazolo[3,2-c]-1,2,4-triazole, 3-(2'-hydroxyéthyl)pyrazolo[3,2-c]-1,2,4-triazole, 6-méthyl-3-éthylpyrazolo[3,2-c]-1,2,4-triazole, 3,6-diméthylpyrazolo[3,2-c]-1,2,4-triazole, 6-méthyl-3-isopropylpyrazolo-[3,2-c]-1,2,4-triazole, 6-méthyl-3-phénylpyrazolo[3,2-c]-1,2,4-triazole, 6-méthyl-3-(2'aminoéthyl)pyrazolo[3,2-c]-1,2,4-triazole, 6-méthyl-3-(2'-hydroxyéthyl)pyrazolo[3,2-c]-1,2,4-triazole, 6-méthyl-3-méthyl-thiopyrazolo[3,2-c]-1,2,4-triazole, 6-phényl-3-méthylpyrazolo[3,2-c]-1,2,4-triazole, 6-phényl-3-éthylpyrazolo[3,2-c]-1,2,4-triazole, 6-isopropyl-3-éthylpyrazolo[3,2-c]-1,2,4-triazole, 6-phényl-3-isopropylpyrazolo[3,2-c]-1,2,4-triazole, 6- phényl-3-phénylpyrazolo-[3,2-c]-1,2,4-triazole, 6-phényl-3-(2'aminoéthyl)pyrazolo[3,2-c]-1,2,4-triazole, 6-phényl-3-(2'-hydroxyéthyl)pyrazolo-[3,2-c]-1,2,4-triazole, 6-phényl-3-méthylthiopyrazolo[3,2-c]-1,2,4-triazole, 6-éthylthio-3-méthylpyrazolo[3,2-c]-1,2,4-triazole, 6-éthylthio-3-éthylpyrazolo[3,2-c]-1,2,4-triazole, 6-éthylthio-3-isopropylpyrazolo[3,2-c]-1,2,4-triazole, 6-éthylthio-3-phénylpyrazolo[3,2-c]-1,2,4-triazole, 6-éthylthio-3(2'-aminoéthyl)pyrazolo[3,2-c]-1,2,4-triazole, 6-éthylthio-3-(2'-hydroxyéthyl)pyrazolo[3,2-c]-1,2,4-triazole, 6-trifluorométhyl-3-méthylthiopyrazolo[3,2-c]-1,2,4-triazole, 6-trifluorométhylpyrazolo[3,2-c]-1,2,4-triazole, 6-carboxy-3-méthylpyrazolo[3,2-c]-1,2,4-triazole, 6-carboxy-3-éthylpyrazolo[3,2-c]-1,2,4-triazole, 6-carboxy-3-isopropylpyrazolo-[3,2-c]-1,2,4-triazole, 6-carboxy-3-phénylpyrazolo[3,2-c]-1,2,4-triazole, 6-carboxy-3-(2'aminoéthyl)pyrazolo[3,2-c]-1,2,4-triazole, 6-carboxy-3-(2'-hydroxyéthyl)pyrazolo[3,2-c]-1,2,4-triazole, 7-chloro-3,6-diméthylpyrazolo[3,2-c]-1,2,4-triazole, 7-méthoxycarbonyl-3,6-diméthylpyrazolo[3,2-c]-1,2,4-triazole, et leurs sels d'addition.

De manière préférée, les pyrazolotétrazoles sont choisis parmi les pyrazolo[5,1-e]tétrazole, 6-méthylpyrazolo[5,1-e]tétrazole, 6-phénypyrazolo[5,1-e]tétrazole, 6-carboxypyrazolo[5,1-e]tétrazole, 7-chloro-6-méthylpyrazolo[5,1-e]tétrazole, et leurs sels d'addition.

De manière préférée, les pyrazolo[1,5-a]imidazoles sont choisis parmi les pyrazolo[1,5-a]imidazole, 2-méthylpyrazolo[1,5-a]imidazole, 2-phénylpyrazolo[1,5-a]imidazole, pyrazolo[1,5-a]benzimidazole, 6-méthylpyrazolo[1,5-a]imidazole, 2,6-diméthylpyrazolo[1,5-a]-imidazole, 6-méthyl-2-phénylpyrazolo[1,5-a]imidazole, 6-méthylpyrazolo[1,5-a]benzimidazole, 6-phénylpyrazolo[1,5-a]-imidazole, 6-phényl-2-méthylpyrazolo[1,5-a]imidazole, 2,6-diphénylpyrazolo[1,5-a]imidazole, 6-phénylpyrazolo[1,5-a]-benzimidazole, 6-carboxypyrazolo[1,5-a]imidazole, 6- carboxy-2-méthylpyrazolo[1,5-a] imidazole, 6-carboxy-2-phénylpyrazolo[1,5-a]-imidazole, 6-carboxypyrazolo[1,5-a]benzimidazole, 6-éthoxypyrazolo-[1,5-a]imidazole, 6-éthoxy-2-méthylpyrazolo[1,5-a]imidazole, 6-éthoxy-2-phénylpyrazolo[1,5-a]imidazole, 6-trifluorométhylpyrazolo-[1,5-a]benzimidazole, 6-aminopyrazolo[1,5-a]imidazole, 6-amino-2-méthylpyrazolo[1,5-a]imidazole, 6-amino-2-phénylpyrazolo[1,5-a]-imidazole, 6-aminopyrazolo[1,5-a]benzimidazole, 6-éthylthiopyrazolo-[1,5-a] imidazole, 6-éthylthio-2-méthylpyrazolo[1,5-a]imidazole, 6-éthylthio-2-phénylpyrazolo[1,5-a]imidazole, 7-chloro-6-méthylpyrazolo[1,5-a]imidazole, 7-chloro-6-méthylpyrazolo[1,5-a]-benzimidazole, et leurs sels d'addition.

De manière préférée, les pyrazolo[5,1-e]pyrazoles sont choisis parmi le 8-amino-4-méthylpyrazolo[5,1-e]pyrazole, le 8-amino-5-chloro-4-méthylpyrazolo[5,1-e]pyrazole, et leurs sels d'addition.

De manière préférée, les pyrazolo[5,1-e]-1,2,3-triazoles sont choisis parmi les 5-méthylpyrazolo[5,1-e]-1,2,3-triazole, 5-méthyl-6-chloropyrazolo[5,1-e]-1,2,3-triazole, 5-phénylpyrazolo[5,1-e]-1,2,3-triazole, et leurs sels d'addition.

Ces pyrazoloazoles sont décrits dans la demande de brevet WO 97/35551.

A titre de pyrroloazoles utilisés dans les compositions selon l'invention, on peut citer les pyrrolo[1,2-b]-1,2,4-triazoles, pyrrolo[2,1-c]-1,2,4-triazoles, pyrrolo[1,2-c]imidazoles, pyrrolo-[1,2-e]tétrazoles, pyrrolo[1,2-a]pyrroles, pyrrolo[1,2-a]imidazoles, pyrrolo[1,2-c]-1,2,3-triazoles, et leurs sels d'addition.

De manière préférée, les pyrrolo[1,2-b]-1,2,4-triazoles sont choisis parmi les 3,4-dicyano-8-méthylpyrrolo[1,2-b]-1,2,4-triazole, 3,4-dicyano-8-phénylpyrrolo[1,2-b]-1,2,4-triazole, 3,4-dicyano-8-tertbutylpyrrolo[1,2-b]-1,2,4-triazole, 5-chloro-3,4-dicyano-8-méthylpyrrolo[1,2-b]-1,2,4-triazole ainsi que les 5-cyano-4-éthoxycarbonyl-8-méthylpyrrolo[1,2-b] 1,2,4-triazole, 5-cyano-4-carboxy-8-méthylpyrrolo[1,2-b]-1,2,4-triazole, 4,5-dicyano-8-méthylpyrrolo[1,2-b]-1,2,4-triazole, 5-cyano-8-méthyl-4-phénylpyrrolo[1,2-b]-1,2,4-triazole, 4,8-diméthylpyrrolo[1,2-b]-1,2,4-triazole, 4,5-di(éthoxycarbonyl)-8-méthylpyrrolo[1,2-b]-1,2,4-triazole, 3-chloro-5-cyano-4-éthoxycarbonyl-8-méthylpyrrolo[1,2-b]-1,2,4-triazole, 5-cyano-4-éthoxycarbonyl-8-phénylpyrrolo[1,2-b]-1,2,4-triazole, 5-cyano-4-carboxy-8-phénylpyrrolo[1,2-b]-1,2,4-triazole, 4,5-dicyano-8-phénylpyrrolo-[1,2-b]-1,2,4-triazole, 4,5-di(éthoxycarbonyl)-8-phénylpyrrolo[1,2-b]-1,2,4-triazole, 3-chloro-5-cyano-4-éthoxycarbonyl-8-phénypyrrolo-[1,2-b]-1,2,4-triazole, 4-cyano-5-carboxy-8-(2-nitro-5-hydroxyphényl)-pyrrolo[1,2-b]-1,2,4-triazole, et leurs sels d'addition.

De manière préférée, les pyrrolo[2,1-c]-1,2,4-triazoles sont choisis parmi les 5,6-dicyano-3-méthylpyrrolo[2,1-c]-1,2,4-triazole, 7-chloro-5,6-dicyano-3-méthylpyrrolo[2,1-c]-1,2,4-triazole, ainsi que les 6,7-dicyano-3-méthylpyrrolo[2,1-c]-1,2,4-triazole, 5-chloro-6,7 dicyano-3-méthylpyrrolo[2,1-c]-1,2,4-triazole, 6,7-di(éthoxycarbonyl)-3-méthylpyrrolo[2,1-c]-1,2,4-triazole, 7-cyano-3-méthyl-6-phényl-pyrrolo[2,1-c]-1,2,4-triazole, 7-cyano-3-méthyl-6-tertbutylpyrrolo-[2,1-c]-1,2,4-triazole, et leurs sels d'addition.

De manière préférée, les pyrrolo[1,2-c]imidazoles sont choisis parmi les 6,8-dicyano-5-éthoxycarbonylpyrrolo[1,2-c]imidazole, 4-chloro-6,8-dicyano-5-éthoxycarbonylpyrrolo[1,2-c]imidazole, et leurs sels d'addition.

De manière préférée, les pyrrolo[1,2-e]tétrazoles sont choisis parmi les 6,7-dicyanopyrrolo[1,2-e]tétrazole, 6-cyano-7-éthoxycarbonylpyrrolo[1,2-e]tétrazole, 5-chloro-6,7-dicyanopyrrolo-[1,2-e]tétrazole, et leurs sels d'addition.

De manière préférée, les pyrrolo[1,2-a]imidazoles sont choisis parmi les 2,3,7-tricyano-6-méthylpyrrolo[1,2-a]imidazole, 2,3,7-tricyano-6-trifluorométhylpyrrolo[1,2-a]imidazole, 2,3,7-tricyano-6-tertbutylpyrrolo[1,2-a]imidazole, 2,3,7-tricyano-6-phénylpyrrolo-[1,2-a]imidazole, 2,3,7-tricyano-6-éthoxycarbonylpyrrolo[1,2-a]-imidazole, 5-chloro-2,3,7-tricyano-6-tertbutylpyrrolo[1,2-a]imidazole, 5-chloro-2,3,7-tricyano-6-phénylpyrrolo[1,2-a]imidazole, 7-cyano-6-éthoxycarbonylpyrrolo[1,2-a]benzimidazole, 7-cyano-6-phénylpyrrolo-[1,2-a]benzimidazole, 7-amido-6-éthoxycarbonypyrrolo[1,2-a]-benzimidazole, et leurs sels d'addition.

De manière préférée, les pyrrolo[1,2-c]-1,2,3-triazoles sont choisis parmi les 5,6,8-tricyanopyrrolo[1,2-c]-1,2,3-triazole, 5,8-dicyano-6-éthoxycarbonylpyrrolo[1,2-c]-1,2,3-triazole, 4-chloro-5,8-dicyano-6-éthoxycarbonylpyrrolo[1,2-c]-1,2,3-triazole, et leurs sels d'addition.

Ces pyrroloazoles sont décrits dans la demande de brevet WO 97/35554.

A titre d'imidazoloazoles utilisés dans les compositions selon l'invention, on peut citer les imidazolo[3,2-a] imidazoles, imidazolo[1,2-b]-1,2,4-triazoles et les imidazolo[2,1-c]-1,2,4-triazoles, et leurs sels d'addition.

De manière préférée, les imidazolo[3,2-a] imidazoles sont choisis parmi les 7,8-dicyano-imidazolo[3,2-a]imidazole, 7,8-dicyano-4-méthylimidazolo[3,2-a]imidazole, 7,8-dicyano-4-éthyl-imidazolo-[3,2-a]imidazole, 7,8-dicyano-4-isopropylimidazolo[3,2-a]imidazole, 7,8-dicyano-4-phénylimidazolo[3,2-a]imidazole, 5-chloro-7,8-dicyano-4-méthylimidazolo[3,2-a]imidazole, 7,8-dicyano-4-trifluorométhylimidazolo[3,2-a]imidazole, et leurs sels d'addition.

De manière préférée, les imidazolo[1,2-b]-1,2,4-triazoles sont choisis parmi les imidazolo[1,2-b]-1,2,4-triazole, 6-méthylimidazolo-[1,2-b]-1,2,4-triazole, 6-isopropylimidazolo[1,2-b]-1,2,4-triazole, 6-phénylimidazolo[1,2-b]-1,2,4-triazole, 2,6-diméthylimidazolo[1,2-b]-1,2,4-triazole, 6-isopropyl-2-méthylimidazolo[1,2-b]-1,2,4-triazole, 2-méthyl-6-phénylimidazolo[1,2-b]-1,2,4-triazole, 6-méthyl-2-phénylimidazolo[1,2-b]-1,2,4-triazole, 6-isopropyl-2-phénylimidazolo[1,2-b]-1,2,4-triazole, 7-chloro-2,6-diméthylimidazolo[1,2-b]-1,2,4-triazole, 7-chloro-2-phényl-6-tertbutylimidazolo[1,2-b]-1,2,4-triazole, 6-trifluorométhylimidazolo[1,2-b]-1,2,4-triazole, et leurs sels d'addition.

De manière préférée, les imidazolo[2,1-c]-1,2,4-triazoles sont choisis parmi les imidazolo[2,1-c]-1,2,4-triazole, 5-méthyl-imidazolo[2,1-c]-1,2,4-triazole, 5,8-diméthylimidazolo[2,1-c]-1,2,4-triazole, 5-méthyl-8-phénylimidazolo[2,1-c]-1,2,4-triazole, 8-phénylimidazolo[2,1-c]-1,2,4-triazole, 6-chloro-5,8-diméthylimidazolo-[2,1-c]-1,2,4-triazole, et leurs sels d'addition.

Ces imidazoloazoles sont décrits dans la demande de brevet WO 97/35552.

Les thiazoloazoles sont décrits dans la demande de brevet FR-A-2 752 524.

A titre de pyrrolooxazoles utilisés dans les compositions selon l'invention, on peut citer les composés décrits, de manière générale, dans les demandes de brevet FR-A-2 752 522, et leurs sels d'addition.

A titre d'hydroxypyrazolopyrimidines utilisées dans les compositions selon l'invention, on peut citer les hydroxypyrazolo-[1,5-a]pyrimidines et plus particulièrement les 2-hydroxy-5-méthyl-7-éthylpyrazolo[1,5-a]pyrimidine, 2-hydroxy-5,6,7-triméthylpyrazolo-[1,5-a]pyrimidine, 2-hydroxy-5,7-diméthyl-6-éthylpyrazolo[1,5-a]-pyrimidine, 2-hydroxy-7-méthylpyrazolo[1,5-a]pyrimidine, 2-hydroxy-5-méthyl-7-carboxypyrazolo[1,5-a]pyrimidine, 2,7-dihydroxy-5,6-diméthylpyrazolo[1,5-a]pyrimidine et leurs sels d'addition. Ces hydroxypyrazolopyrimidines sont décrites dans la demande de brevet DE-A-40 29 324.

A titre d'isoxazolones utilisées dans les compositions selon l'invention, on peut citer les 4-carboxy-β,γ-benzoisoxazolone, 1-acétyl-4-carboxy-β,γ-benzoisoxazolone, 6-carboxy-β,γ-benzoisoxazolone, 1-acétyl-6-carboxy-β,γ-benzoisoxazolone, β,γ-benzoisoxazolone, 1-acétyl-β,γ-benzoisoxazolone, 4-méthyl-β,γ-benzoisoxazolone, 1-acétyl-4-(β-hydroxyéthylamino)carbonyl-β,γ-benzoisoxazolone, 3-phényl-isoxazol-5-one, 2-acétyl-3-phénylisoxazol-5-one, 3,4-diphénylisoxazol-5-one, 3-méthylisoxazol-5-one, 3,4-tétraméthylèneisoxazol-5-one, et leurs sels d'addition.

Ces isoxazolones sont décrites dans la demande de brevet FR-A-2 040 260.

A titre d'indazolones utilisées dans les compositions selon l'invention, on peut citer les indazolone, 5-chloroindazolone, 6-chloroindazolone, 1-éthylindazolone, 5-diméthylaminoindazolone, 1-méthylindazolone, 1-isopropylindazolone, 1-butylindazolone, 3-chloroindazolone, 4-chloroindazolone, 5-méthylindazolone, 6-méthylindazolone, 5-éthylindazolone, 6-propylindazolone, 5-butylindazolone, 1,5-diméthylindazolone, 1,6-diméthylindazolone, 1-méthyl-5-chloroindazolone, 1-méthyl-6-chloroindazolone, 1-éthyl-5-chloroindazolone, 1-éthyl-6-bromo-indazolone, 5-aminoindazolone, 6-diméthylaminoindazolone, 5-diéthylaminoindazolone, 1-méthyl-5-diméthylaminoindazolone 5-dibutylaminoindazolone, 1-éthyl-5-dipropylaminoindazolone, et leurs sels d'addition.

Ces indazolones sont décrites dans la demande de brevet DE-A-26 32 390.

A titre de benzimidazoles utilisés dans les compositions selon l'invention, on peut citer le 4,7-dihydroxybenzimidazole, la 4,7-dihydroxy-1-méthylbenzimidazole, le 4,7-dihydroxy-2-méthylbenzimidazole, le 4,7-dihydroxy-1-éthylbenzimidazole, le 4,7-dihydroxy-1-propylbenzimidazole, 4,7-dihydroxy-1-butylbenzimidazole, le 4,7-dihydroxy-2-éthylbenzimidazole, le 4,7-dihydroxy-2-butylbenzimidazole, le 4,7-dihydroxy-1,2-diméthylbenzimidazole, le 4,7-diméthoxybenzimidazole, le 4,7-diméthoxy-1-méthylbenzimidazole, le 4,7-diméthoxy-1-éthylbenzimidazole, le 4,7-diméthoxy-2-méthylbenzimidazole, le 4,7-diméthoxy-2-éthylbenzimidazole, le 5,6-dihydroxybenzimidazole, le 5,6-dihydroxy-1-méthylbenzimidazole, le 5,6-dihydroxy-1-éthylbenzimidazole, le 5,6-dihydroxy-1-butylbenzimidazole, le 5,6-dihydroxy-2-méthybenzimidazole, le 5,6-dihydroxy-2-butylbenzimidazole, le 5,6-dihydroxy-2-phénylbenzimidazole, le 5,6-diméthoxybenzimidazole, le 5,6-diméthoxy-1-méthylbenzimidazole, le 5,6 diméthoxy-1-éthylbenzimidazole, le 5,6-diméthoxy-1-propylbenzimidazole, le 5,6-diméthoxy-2-méthylbenzimidazole, le 5,6-diméthoxy-2-butylbenzimidazole, le 5,6-diméthoxy-2-phénylbenzimidazole, le 5,6-diméthoxy-1,2-diméthylbenzimidazole, le 4-hydroxy-7-méthoxybenzimidazole, le 5-hydroxy-6-méthoxybenzimidazole, le 4-hydroxy-7-méthoxy-1-méthylbenzimidazole, le 5-hydroxy-6-méthoxy-1,2-diméthylbenzimidazole. Ces benzimidazoles sont décrits dans la demande de brevet DE-A-28 12 678.

A titre de benzimidazoles utilisés dans les compositions selon l'invention, on peut encore citer les ω-cyanoacétylbenzimidazoles, décrits de manière générale dans la demande DE-A-24 46 632, et en particulier le 5-amino-1-méthyl-2-(ω-cyanoacétyl)benzimidazole, ainsi que leurs sels d'addition.

A titre de coupleurs pyridiniques utilisés dans les compositions selon l'invention, on peut citer la 2-amino-3-hydroxypyridine, les 2,3-diaminopyridines, les 3-amino-5-hydroxypyridines, et leurs sels d'addition.

A titre de 2,3-diaminopyridines utilisées dans les compositions selon l'invention, on peut citer les 6-méthoxy-3-amino-2-phényl-aminopyridine, 6-méthoxy-3-amino-2-(4'-hydroxyphényl)pyridine, 6-méthoxy-3-amino-2-(2'-méthoxyphényl)aminopyridine, 6-méthoxy-3-amino-2-(2'-hydroxyphényl)aminopyridine, 6-méthoxy-3-amino-2-diéthylaminopyridine, 6-méthoxy-3-amino-2-diméthylaminopyridine, le 6-méthoxy-3-amino-2-(méthyl, 2'-hydroxyéthyl)aminopyridine, 6-méthoxy-3-amino-2-(n-butyl, 2'-hydroxyéthyl)pyridine, 6-méthoxy-3-amino-2-bis-(2'-hydroxyéthyl)aminopyridine, 6-méthoxy-3-amino-2-(2',3'-dihydroxypropyl)aminopyridine, 6-méthoxy-3-amino-2-(1',1'-diméthyl-2'-hydroxyéthyl)aminopyridine, 6-méthoxy-3-amino-2-(1'-hydroxyméthyl-2'-hydroxyéthyl)aminopyridine, 6-méthoxy-3-amino-2-(1'-méthyl-2'-hydroxyéthyl)aminopyridine, 6-méthoxy-3-amino-2-(3'-diméthylaminopropyl)aminopyridine, 6-méthoxy-3-amino-2-bis-(méthoxyéthyl)aminopyridine, 6-méthoxy-3-amino-2-bis-(2'-propényl)-aminopyridine, 6-méthoxy-3-amino-2-pyrrolidinylpyridine, 6-méthoxy-3-amino-2-(3'-acétamidopyrrolidinyl)pyridine, 6-méthoxy-3-amino-2-(2',5'-diméthylpyrrolidinyl)pyridine, 6-méthoxy-3-amino-2-(2'-diméthylaminoéthyl)aminopyridine, 6-méthoxy-3-amino-2-morpholinopyridine, 6-méthoxy-3-amino-2-(2'-méthylpyrrolidinyl)pyridine, 6-méthoxy-3-amino-2-pipérazinylpyridine, 6-méthoxy-3-amino-2-pyridinylpyridine, 6-méthoxy-3-amino-2-pyrrolidinylpyridine, 6-méthoxy-3-amino-2-(2'-méthylpyridinyl)pyridine, 6-méthoxy-3-amino-2-(2'-hydroxyéthylpyridinyl)pyridine, 6-méthoxy-3-amino-2-(2'-pyrrolidinyléthyl)aminopyridine, 6-méthoxy-3-amino-2-(3'-imidazolin-ylpropyl)aminopyridine, 6-méthoxy-3-amino-2-(3'(3"-méthyl-imidazolium)propyl)aminopyridine, 6-(2'-trifluoroéthoxy)-5-trifluorométhyl-2,3-diaminopyridine, 6-phénoxy-5-trifluorométhyl-2,3-diaminopyridine, la 6-méthoxy-2,3-diaminopyridine, et leurs sels d'addition.

De préférence, parmi ces derniers composés, le coupleur pyridinique est choisi parmi les composés 6-méthoxy-3-amino-2-hydroxyéthylaminopyridine, 6-méthoxy-3-amino-2-(2',3'-dihydroxypropyl)aminopyridine, 6-méthoxy-3-amino-2-(1'-méthyl-2'-hydroxyéthyl)aminopyridine, 6-méthoxy-3-amino-2-pyrrolidinylpyridine, 6-méthoxy-3-amino-2-(2'-méthylpyrrolidinyl)pyridine, 6-méthoxy-3-amino-2-(2'-méthylpyridinyl)pyridine, 6-méthoxy-3-amino-2-(2'-hydroxyéthylpyridinyl)pyridine, la 6-méthoxy-2,3-diaminopyridine, et leurs sels d'addition.

Ces coupleurs peuvent être peuvent être préparés selon des méthodes connues et décrites dans la littérature. On pourra se reporter à titre d'exemples à la demande de brevet DE-A-32 33 540.

A titre de 3-amino-5-hydroxypyridines utilisées dans les compositions selon l'invention, on peut citer la 3-amino-5-hydroxy-2,6-diméthoxypyridine, la 3-amino-5-hydroxy-2,6-di-(2'-hydroxyéthyloxy)-pyridine, et leurs sels d'addition. Ces 3-amino-5-hydroxypyridines sont décrites dans la demande de brevet DE-A-34 42 128.

A titre de coupleurs pyridiniques, on utilisera, de préférence, la 2-amino-3-hydroxypyridine et ses sels d'addition.

A titre de thiophènes utilisés dans les compositions selon l'invention, on peut citer les ω-cyanoacétylthiophènes, décrits de manière générale dans la demande DE-A-24 46 632, et en particulier le 5-amino-2-(ω-cyanoacétyl)thiophène, ainsi que leurs sels d'addition.

A titre d'indolines utilisées dans les compositions selon l'invention, on peut citer les 5-aminoindolines, les 6-aminoindolines, les 7-aminoindolines, la 4-hydroxyindoline, la 5-hydroxyindoline, la 6-hydroxyindoline, la 5,6-dihydroxyindoline, les 5,6-diaminoindoline et 5,7-diaminoindoline, la 5-amino-6-nitroindoline, la 5-bromo-7-nitroindoline, la 6-nitroindoline, et leurs sels d'addition, et notamment, leurs chlorhydrates. Ces indolines sont décrites dans le brevet US 4,013,404.

Parmi les 5,7-diaminoindolines, on peut citer : la 5,7-diamino-1-méthylindoline, la 5,7-diamino-2-méthylindoline, la 5,7-diamino-3-méthylindoline, la 5,7-diamino-2,2-diméthylindoline, la 5,7-diamino-2,3-diméthylindoline, la 5,7-diamino-2-méthyl-3-éthylindoline, la 5,7-diamino-1-éthyl-2-méthyl-2-éthylindoline, la 5,7-diamino-6-méthylindoline, la 5,7-diamino-1,6-diméthylindoline, la 5-diméthylamino-7-amino-1-butylindoline, la 5-diéthylamino-7-amino-2,2-dipropylindoline, la 5-amino-7-diméthylamino-2-méthyl-3-butylindoline, 5-amino-7-dibutylamino-3,3-diéthylaminoindoline, la 5,7-bis-diméthylaminoindoline, et leurs sels d'addition. Ces indolines sont décrites dans la demande de brevet DE-A-27 16 671.

On peut également citer les indolines suivantes et leurs sels : la 6-aminoindoline, la 6-hydroxyindoline, la 1-éthyl-6-aminoindoline, la 1-N-éthyl-4-hydroxyindoline. Ces indolines sont décrites dans la demande de brevet DE-A-19 16 139.

Parmi les 5,6-dihydroxyindolines, on peut citer : la 5,6-dihydroxyindoline, la N-méthyl-5,6-dihydroxyindoline, la N-éthyl-5,6-dihydroxyindoline, la N-propyl-5,6-dihydroxyindoline, la N-butyl-5,6-dihydroxyindoline et la 2-carboxy-5,6-dihydroxyindoline, et leurs sels d'addition. Ces indolines sont décrites dans la demande de brevet WO 01/93818.

A titre d'indoles utilisés dans les compositions selon l'invention, on peut citer le 6-hydroxyindole et ses dérivés, le 5,6-dihydroxyindole et ses dérivés, le 4-hydroxyindole et ses dérivés, ainsi que leurs sels d'addition. De préférence, le coupleur indolique est le 6-hydroxyindole.

A titre de benzofuranes utilisés dans les compositions selon l'invention, on peut citer les hydroxybenzofuranes, les diaminobenzofuranes et les ω-cyanoacétylbenzofuranes, et leurs sels d'addition.

De manière préférée, les hydroxybenzofuranes utilisés sont les 2-méthyl-6-hydroxybenzofurane, 3-méthyl-6-hydroxybenzofurane, 2,4-diméthyl-6-hydroxybenzofurane, 3-n-propyl-6-hydroxybenzofurane, 2-éthyl-5-hydroxybenzofurane, 2-méthyl-5-hydroxybenzofurane, 3-méthyl-5-hydroxybenzofurane, le 3-isobutyl-5-hydroxybenzofurane, 3-éthyl-5-hydroxybenzofurane, 2,6-diméthyl-5-hydroxybenzofurane, 3,6-diméthyl-5-hydroxybenzofurane, 6,7-diméthyl-5-hydroxybenzofurane, 3-n-propyl-5-hydroxybenzofurane, 3-méthyl-4-n-propyl-5-hydroxybenzofurane, 2-hexyl-5-hydroxybenzofurane, 2-n-propyl-5-hydroxybenzofurane, 4-tertiobutyl-5-hydroxybenzofurane, 6-tertiobutyl-5-hydroxybenzofurane, 4-méthyl-5-hydroxybenzofurane, 3-méthyl-5-n-propyl-4-hydroxybenzofurane, 2-éthyl-4-hydroxybenzofurane, 2-méthyl-6-pentyl-4-hydroxybenzofurane, 6-pentyl-4-hydroxybenzofurane, 3,5-diméthyl-4-hydroxybenzofurane, 3,7-diméthyl-4-hydroxybenzofurane, 2,6-di-tertiobutyl-4-hydroxybenzofurane, 2-méthyl-4-hydroxybenzofurane, 3-méthyl-4-hydroxybenzofurane, 2-méthyl-7-éthyl-4-hydroxybenzofurane, 2,7-diméthyl-4-hydroxybenzofurane, 2-isopropyl-4-hydroxybenzofurane, 3-éthyl-4-hydroxybenzofurane, 3-méthyl-7-tertiobutyl-4-hydroxybenzofurane, 3-méthyl-5-tertiobutyl-4-hydroxybenzofurane, 2,6-diméthyl-4-hydroxybenzofurane, 3-isopropyl-4-hydroxybenzofurane, 3-n-propyl-4-hydroxybenzofurane, 3-méthyl-7-n-propyl-4-hydroxybenzofurane, 3-méthyl-6-n-propyl-7-hydroxybenzofurane, 3-méthyl-7-hydroxybenzofurane, 2-éthyl-4-méthyl-7-hydroxybenzofurane, 2-éthyl-5-méthyl-7-hydroxybenzofurane, et leurs sels d'addition. Ces hydroxybenzofuranes sont décrits dans la demande de brevet EP-A-0 506 549.

De manière préférée, les diaminobenzofuranes utilisés sont les 5,7-diaminobenzofurane, 5,7-diamino-2-méthylbenzofurane, 5,7-diamino-2-éthylbenzofurane, 5-diméthylamino-7-aminobenzofurane, 4,6-diaminobenzofurane, et leurs sels d'addition. Ces diaminobenzofuranes sont décrits dans la demande de brevet DE-A-27 19 424.

De manière préférée, les ω-cyanoacétylbenzofuranes utilisés sont les ω-cyanoacétylbenzofuranes décrits de manière générale dans la demande DE-A-24 46 632, et en particulier le 5-amino-2-(ω-cyanoacétyl)benzofurane, ainsi que leurs sels d'addition.

A titre de 8-amino-6-méthoxyquinoléines utilisées dans les compositions selon l'invention, on peut citer les 8-amino-6-méthoxyquinoléine, 8-amino-5-bromo-6-méthoxyquinoléine, 8-amino-5-chloro-6-méthoxyquinoléine, 8-amino-5,7-dibromo-6-méthoxyquinoléine, 8-amino-5-méthyl-6-méthoxyquinoléine, 8-amino-5,7-diméthyl-6-méthoxyquinoléine, 8-amino-5-éthyl-6-méthoxyquinoléine, 8-amino-5-butyl-6-méthoxyquinoléine, 8-amino-5-phényl-6-méthoxyquinoléine, 8-amino-2-phényl-6-méthoxyquinoléine, 8-amino-2-benzyloxy-6-méthoxyquinoléine, 8-amino-4-diméthylamino-6-méthoxyquinoléine, 8,4-diamino-6-méthoxyquinoléine, 8-amino-4-chloro-6-méthoxyquinoléine, et leurs sels d'addition. Ces 8-amino-6-méthoxyquinoléines sont décrites dans la demande de brevet DE-A-26 26 141.

A titre de 4-hydroxyquinolones utilisées dans les compositions selon l'invention, on peut citer les 7-diméthylamino-4-hydroxy-2-quinolone, 6-méthyl-4-hydroxy-2-quinolone, 6-diméthylamino-4-hydroxy-2-quinolone, 6-méthoxy-4-hydroxy-2-quinolone, 8-chloro-4-hydroxy-2-quinolone, 1-méthyl-7-diméthylamino-4-hydroxy-2-quinolone, 1-méthyl-4-hydroxy-2-quinolone, 1-méthyl-8-chloro-4-hydroxy-2-quinolone, 1,6-diméthyl-4-hydroxy-2-quinolone, 1-méthyl-6-diméthylamino-4-hydroxy-2-quinolone, 6-(2-hydroxyéthyl)-4-hydroxy-2-quinolone, 1-isopropyl-4-hydroxy-2-quinolone, 1-méthyl-7-isopropyl-4-hydroxy-2-quinolone, 1-n-butyl-8-bromo-4-hydroxy-2-quinolone, et leurs sels d'addition. Ces 4-hydroxyquinolones sont décrites dans la demande de brevet DE-A-23 34 738.

A titre de benzodioxoles utilisés dans les compositions selon l'invention, on peut citer les composés décrits, de manière générale, dans les demandes de brevet DE-A-197 18 534 et DE-A-28 13 076.

De manière préférée, les benzodioxoles utilisés sont les 5-amino-1,3-benzodioxole, 5-hydroxy-1,3-benzodioxole, 5-amino-2-méthyl-1,3-benzodioxole, 5-hydroxy-2,2-diméthyl-1,3-benzodioxole, 5-hydroxy-2-éthyl-1,3-benzodioxole, 5-hydroxy-2-butyl-1,3-benzodioxole, 5-hydroxy-2-phényl-1,3-benzodioxole, 5,6-dihydroxy-1,3-benzodioxole, 4,7-dihydroxy-1,3-benzodioxole, 4,7-diamino-2-méthyl-1,3-benzodioxole, 5,6-diamino-2,2-diphényl-1,3-benzodioxole, 4,5,7-triamino-1,3-benzodioxole, 5-hydroxy-7-méthyl-2,2-diéthyl-1,3-benzodioxole, et leurs sels d'addition avec un acide, décrits dans la demande de brevet DE-A-28 13 076.

A titre d'hydroxybenzamides utilisés dans les compositions selon l'invention, on peut citer les 2,4-dihydroxybenzamides et en particulier les N-phényl-2,4-dihydroxybenzamide, N-(2'-méthoxyphényl)-2,4-dihydroxybenzamide, N-(3'-méthoxyphényl)-2,4-dihydroxybenzamide, N-(4'-méthoxyphényl)-2,4-dihydroxybenzamide, N-(4'-carboxyphényl)-2,4-dihydroxybenzamide, N-(2'-pyridyl)-2,4-dihydroxybenzamide, N-(3'-pyridyl)-2,4-dihydroxybenzamide, N-(2',5'-diméthoxyphényl)-2,4-dihydroxybenzamide, N-(3',5'-diméthoxyphényl)-2,4-dihydroxybenzamide, N-(2'-méthoxy-5'-aminophényl)-2,4-dihydroxybenzamide, N-(4'-(N,N-diméthylamino)phényl)-2,4-dihydroxybenzamide, N-(4'-hydroxyphényl)-2,4-dihydroxybenzamide, N-méthyl-2,4-dihydroxybenzamide, N-benzyl-2,4-dihydroxybenzamide, le 2,4-dihydroxybenzamide non substitué, et leurs sels d'addition. Ces hydroxybenzamides sont décrits dans la demande de brevet DE-A-38 22 449.

A titre de dérivés de sésamol utilisés dans les compositions selon l'invention, on peut citer outre le sésamol, le 1-N-β-hydroxyéthylamino-3,4-méthylènedioxybenzène.

A titre de benzomorpholines utilisées dans les compositions selon l'invention, on peut citer la 6-hydroxybenzomorpholine et la 6-aminobenzomorpholine, et leurs sels d'addition.

A titre de coupleur(s) naphtalénique(s) utilisable(s) dans les compositions selon l'invention, on peut citer l'alpha-naphtol, les naphtalènes substitués de formule (VIII) suivante, et leurs sels d'addition : 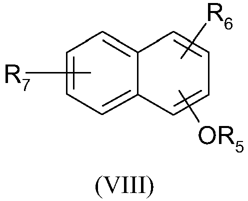 dans laquelle :
R₅ représente un atome d'hydrogène ou un groupement -CO-R dans lequel R représente un groupe alkyle en C₁-C₄ ;
R₆ représente un atome d'hydrogène, un groupe hydroxyle, un groupe alkyle en C₁-C₄ ou un groupe -SO₃H ;
R₇ représente un atome d'hydrogène, ou un groupe hydroxyle ;
étant entendu qu'au moins un des groupes R₅ à R₇ est différent d'un atome d'hydrogène.

Parmi les coupleurs naphtaléniques utilisables de manière préférée dans les compositions tinctoriales conformes à l'invention, on peut notamment citer :
- l'alpha-naphtol,
- le 1,7-dihydroxynaphtalène,
- le 2,7-dihydroxynaphtalène,
- le 2,5-dihydroxynaphtalène,
- le 2,3-dihydroxynaphtalène,
- le 1-acétoxy-2-méthylnaphtalène,
- le 1-hydroxy-2-méthylnaphtalène,
- l'acide 1-hydroxy-4-naphtalène sulfonique,
et leurs sels d'addition.

De manière particulièrement préférée, les coupleurs d'oxydation utilisés dans les compositions selon l'invention sont choisis parmi les méta-aminophénols, les méta-phénylènediamines, les méta-diphénols, les indolines et les indoles, ainsi que leurs sels d'addition.

De préférence, le (ou les) colorant(s) d'oxydation de l'invention est (ou sont) choisi(s) parmi les colorants d'oxydation benzéniques ou hétérocycliques.

Les bases d'oxydation et coupleurs d'oxydation peuvent être présents dans les compositions de l'invention, sous forme de sels d'addition et, en particulier sous forme de sels d'addition avec un acide.

Les sels d'addition avec un acide utilisables dans le cadre de l'invention sont, notamment, choisis parmi les chlorhydrates, les bromhydrates, les sulfates, les citrates, les succinates, les tartrates, les lactates, les acétates, les alkylsulfates et les alkylsulfonates.

Lorsque les bases d'oxydation ou les coupleurs d'oxydation contiennent une ou plusieurs fonctions acide carboxylique ou sulfonique, les sels d'addition avec une base sont envisageables.

Les sels d'addition avec une base utilisables dans le cadre des compositions tinctoriales de l'invention sont alors notamment ceux obtenus avec de la soude, de la potasse, de l'ammoniaque ou des amines.

La concentration en colorant(s) d'oxydation des compositions selon l'invention va de préférence de 0,005 à 15 % en poids, en particulier de 0,01 à 10 % en poids, et de manière plus préférée de 0,5 à 5 % en poids, par rapport au poids total de la composition.

La composition tinctoriale conforme à l'invention peut, en outre, contenir un ou plusieurs colorant(s) direct(s) pouvant notamment être choisi(s) parmi les colorants nitrés benzéniques, les colorants directs azoïques, les colorants directs méthiniques, et leurs sels d'addition. Ces colorants directs peuvent être de nature non ionique, anionique ou cationique.

Le milieu utilisé dans les compositions selon la présente invention est un milieu aqueux ou un milieu contenant de l'eau et au moins un solvant organique.

Le (ou les) solvant(s) organique(s) utilisé(s) dans les compositions selon la présente invention peut (ou peuvent) être choisi(s) parmi les alcools monohydroxylés et les polyols.

A titre d'alcools monohydroxylés utilisables, on peut citer les alcools inférieurs en C₁-C₄ comme l'éthanol, l'isopropanol, le tertiobutanol, le n-butanol, et leurs mélanges. De préférence, l'alcool utilisé est l'éthanol.

À titre de polyols utilisables, on peut citer le propylèneglycol, les polyéthylèneglycols, les éthers de polyols comme le 2-butoxyéthanol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

La concentration en solvant(s) organique(s) dans les compositions selon la présente invention est comprise de préférence entre 0 et 30 % en poids, et de manière plus préférée entre 0 et 20 % en poids, par rapport au poids total de la composition.

La composition tinctoriale conforme à l'invention peut également contenir un ou plusieurs adjuvant(s) utilisé(s) classiquement dans les compositions pour la teinture des cheveux, tel(s) que les agents tensioactifs anioniques, cationiques, non-ioniques, amphotères, zwittérioniques ou leurs mélanges ; les polymères non-ioniques, amphotères, zwittérioniques, anioniques, cationiques additionnels - autres que les éthers de cellulose cationiques selon l'invention -, ou leurs mélanges ; les agents de pénétration ; les agents séquestrants ; les parfums ; les tampons ; les agents dispersants ; les agents conditionneurs tels que par exemple des silicones volatiles ou non volatiles, modifiées ou non modifiées ; les agents filmogènes ; les céramides ; les agents conservateurs ; les agents opacifiants ; les vitamines ; les acides aminés ; les oligopeptides ; les peptides ; les protéines hydrolysées ou non, modifiées ou non ; les enzymes ; les acides et alcools gras, ramifiés ou non ; les cires animales, végétales ou minérales ; les acides organiques hydroxylés ; les filtres UV ; les agents anti-oxydants et les agents anti-radicaux libres ; les agents antipelliculaires ; les agents régulateurs de séborrhée ; les agents apaisants ; les huiles minérales, végétales ou animales ; les polyisobutènes et poly(α-oléfines) ; les pigments ; les acides, bases, plastifiants, charges minérales, nacres, paillettes ; les agents antistatiques et les agents réducteurs.

Le (ou les) adjuvant(s) ci-dessus est (ou sont), en général, présent(s) en quantité comprise, pour chacun d'eux, de préférence entre 0,01 et 40 % en poids, , et de manière plus préférée entre 0,1 et 25 % en poids, par rapport au poids de la composition.

Les compositions selon la présente demande peuvent également contenir, en tant qu'adjuvant cosmétique additionnel, au moins un agent épaississant encore appelé "agent d'ajustement de la rhéologie".

L'agent (ou les agents) d'ajustement de la rhéologie peut (ou peuvent) être choisi(s) parmi les agents épaississants minéraux ou organiques, et en particulier les épaississants associatifs polymériques, les alcools gras additionnels - autres que les tensioactifs glycérolés selon l'invention - (alcool oléïque), les dérivés cellulosiques additionnels - autre que les éthers de cellulose cationiques selon l'invention - (hydroxyéthylcellulose, hydroxypropylcellulose, carboxyméthyl-cellulose) et les gommes d'origine microbienne (gomme de xanthane, gomme de scléroglucane).

L'agent (ou les agents) d'ajustement de la rhéologie préféré(s) est (ou sont) choisi(s) parmi les alcools gras, les éthers de celluloses non ioniques et les gommes d'origine microbienne.

La concentration en agent(s) épaississant(s) est comprise de préférence entre 0,01 et 20 % en poids, et de manière plus préférée entre 1 et 10 % en poids, par rapport au poids total de la composition.

Bien entendu, l'homme de l'art veillera à choisir ce (ou ces) éventuel(s) composé(s) complémentaire(s) de manière telle que les propriétés avantageuses attachées intrinsèquement à la composition de teinture d'oxydation conforme à l'invention ne soient pas, ou substantiellement pas, altérées par la (ou les) adjonction(s) envisagée(s).

Le pH de la composition tinctoriale conforme à l'invention va généralement de 3 à 12 environ, et de préférence entre 5 et 11 environ. Il peut être ajusté à la valeur désirée au moyen du (ou des) métasilicate(s) selon l'invention, éventuellement associé(s) à d'autre(s) agent(s) acidifiant(s) ou alcalinisant(s), habituellement utilisé(s) en teinture des fibres kératiniques ou bien encore à l'aide de système(s) tampon(s) classique(s).

Parmi les agents acidifiants, on peut citer, à titre d'exemple, les acides minéraux ou organiques comme l'acide chlorhydrique, l'acide orthophosphorique, l'acide sulfurique, les acides sulfoniques et les acides carboxyliques comme l'acide acétique, l'acide tartrique, l'acide citrique et l'acide lactique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IX) suivante : 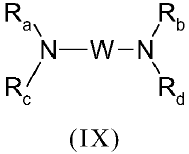 dans laquelle :
W est un reste propylène éventuellement substitué par un groupe hydroxyle ou un groupe alkyle en C₁-C₄ ;
Rₐ, R_{b}, R_{c} et R_{d}, identiques ou différents, représentent un atome d'hydrogène, un groupe alkyle en C₁-C₄ ou hydroxyalkyle en C₁-C₄.

La composition tinctoriale selon l'invention peut se présenter sous des formes diverses, telles que sous forme de crèmes, de gels, ou sous toute autre forme appropriée pour réaliser une teinture des fibres kératiniques, et notamment des cheveux humains.

Le procédé de teinture des fibres kératiniques de la présente invention est un procédé dans lequel on applique sur les fibres la composition selon la présente invention telle que définie précédemment, de préférence, en présence d'au moins un agent oxydant pendant un temps suffisant pour développer la couleur désirée. La couleur peut être révélée à pH acide, neutre ou alcalin et l'agent (ou les agents) oxydant(s) peut (ou peuvent) être ajouté(s) à la composition de l'invention juste au moment de l'emploi ou il(s) peut (ou peuvent) être mis en oeuvre à partir d'une composition oxydante le(s) contenant, appliquée simultanément ou séquentiellement à la composition de l'invention.

Selon un mode de réalisation particulier, la composition selon la présente invention est une composition prête à l'emploi, mélangée, de préférence au moment de l'emploi, à une composition contenant, dans un milieu approprié pour la teinture, au moins un agent oxydant, cet agent (ou ces agents) oxydant(s) étant présent(s) en une quantité suffisante pour développer une coloration. Le mélange obtenu est ensuite appliqué sur les fibres kératiniques. Après un temps de pause de 3 à 50 minutes environ, de préférence, 5 à 30 minutes environ, les fibres kératiniques sont rincées, lavées au shampooing, rincées à nouveau, puis séchées.

Les agents oxydants classiquement utilisés pour la teinture d'oxydation des fibres kératiniques sont, par exemple, le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, ces oxydo-réductases étant éventuellement associées à leurs cofacteurs habituels tels que l'acide urique pour les uricases. L'agent oxydant préféré est le peroxyde d'hydrogène.

La composition oxydante peut également renfermer divers adjuvants utilisés classiquement dans les compositions pour la teinture des cheveux, tels que définis précédemment.

Le pH de la composition oxydante renfermant l'agent oxydant est tel qu'après mélange avec la composition tinctoriale, le pH de la composition résultante appliquée sur les fibres kératiniques varie, de préférence, de 3 à 12 environ, et préférentiellement, de 5 à 10. Il peut être ajusté à la valeur désirée au moyen d'agent(s) acidifiant(s) ou alcalinisant(s) habituellement utilisé(s) en teinture des fibres kératiniques, tel(s) que défini(s) précédemment.

La composition prête à l'emploi qui est finalement appliquée sur les fibres kératiniques, peut se présenter sous des formes diverses, telles que sous forme de crèmes, de gels ou sous toute autre forme appropriée pour réaliser une teinture des fibres kératiniques, et notamment des fibres kératiniques humaines tels que les cheveux.

L'invention a aussi pour objet un dispositif à plusieurs compartiments ou "kit" de teinture comprenant au moins un premier compartiment contenant la composition tinctoriale définie ci-dessus et au moins un deuxième compartiment contenant une composition oxydante. Ce dispositif peut être équipé d'un moyen permettant de délivrer sur les cheveux le mélange souhaité, tel que les dispositifs décrits dans la demande de brevet FR-A-2 586 913.

Les exemples qui suivent servent à illustrer l'invention sans toutefois présenter un caractère limitatif.

### EXEMPLES

Les compositions suivantes ont été réalisées.

| | **Composition 1** | **Composition 2** |
|---|---|---|
| 1-méthyl-2,5-diaminobenzène | 1,7 g | 0,5 g |
| 1-hydroxy-4-aminobenzène | - | 0,4 g |
| 1,3-dihydroxybenzène | 1 g | 0,25 g |
| 1-hydroxy-3-aminobenzène | 0,07 g | - |
| Dichlorhydrate de 1-béta-hydroxyéthyloxy-2,4-diaminobenzène | 0,03 g | - |
| 2-méthyl-1,3-dihydroxybenzène | 0,5 g | 0,3 g |
| 1-méthyl-2-hydroxy-4-aminobenzène | - | 0,25 g |
| 1-méthyl-2-hydroxy-4-béta-hydroxyéthylamino benzène | - | 0,05 g |
| 6-hydroxyindole | - | 0,01 g |
| Métasilicate de sodium | 2 g | 1 g |

| | | |
|---|---|---|
| Monoéthanolamine pure | 5,7 g | 0,7 g |
| Solution aqueuse d'ammoniac à 20 % en poids | - | 4 g |
| Ether de cellulose cationique (Softcat SL-60 vendu par Amerchol) | 0,2 g | 0,5 g |
| Alcools en C₂₀-C₂₂ (Nafol 2022 EN vendu par Sasol) | 3 g | 3 g |
| Alcool oléique | 0,5 g | 0,5 g |
| Monoéthanolamide d'acide laurique | 3 g | 3 g |
| Alcool stéarylique oxyéthyléné à 2 moles d'oxyéthylène | 5 g | 5 g |
| Alcool stéarylique oxyéthyléné à 21 moles d'oxyéthylène | 3,8 g | 3,8 g |
| Acide oléique | 3 g | 3 g |
| Solution aqueuse à 60 % en poids de chlorure d'hexadiméthrine (Mexomère 90 vendu par Chimex) | 2 g | 2 g |
| Solution aqueuse à 40 % en poids de polyquaternium-6 (Merquat 100 vendu par Ondéo) | 4 g | 4 g |
| TiO₂ | 0,2 g | 0,2 g |
| Réducteur, antioxydant, séquestrant, parfum | q.s. | q.s. |
| Eau déminéralisée q.s.p. | 100 g | 100 g |

### Protocole d'application

Chaque composition est diluée extemporanément, avec une fois et demie son poids d'eau oxygénée (pH voisin de 3) à 9 volumes (i.e. 2,7 % en poids d'H₂O₂) pour la composition 1 et à 20 volumes (i.e. 6 % en poids d'H₂O₂) pour la composition 2. Le mélange ainsi réalisé est une crème de bonne consistance qui s'applique facilement sur des cheveux gris, à 90 % de cheveux blancs, à raison de 10 g pour 1 g de cheveux, pendant 20 minutes. Les cheveux sont ensuite facilement rincés, lavés avec un shampooing standard et séchés.

La coloration capillaire est évaluée de manière visuelle. Les résultats obtenus sur les cheveux naturels gris, à 90 % de cheveux blancs, après traitement, sont les suivants :

| | **Nuance** |
|---|---|
| **Composition 1** | Châtain naturel |
| **Composition 2** | Blond foncé cuivré acajou |

Ces colorations possèdent de bonnes propriétés notamment en termes de sélectivité et de ténacité. Elles possèdent aussi une bonne intensité. Les compositions obtenues sont stables dans le temps.

Les compositions suivantes ont également été réalisées :

| | **Composition 3** | **Composition 4** |
|---|---|---|
| 1-méthyl-2,5-diaminobenzène | - | 2 g |
| Hydrochlorure de 2-(4,5-diamino-1H-pyrazollyl)éthanol | 2,15 g | - |
| 1-hydroxy-3-aminobenzène | 1,1 g | 0,5 g |
| Monohydrate de sulfate de N,N-bis(2-hydroxyéthyl)-para-phénylènediamine | - | 0,4 g |
| 1,3-dihydroxybenzène | - | 1,2 g |
| Dichlorhydrate de 1-béta-hydroxyéthyloxy-2,4-diaminobenzène | - | 0,6 g |
| 6-hydroxybenzomorpholine | - | 0,15 g |
| Métasilicate de sodium | 2 g | 1 g |
| Monoéthanolamine pure | 5,7 g | 5,92 g |
| Solution aqueuse d'ammoniac à 20 % en poids | - | 4 g |
| Ether de cellulose cationique (Softcat SL-100 vendu par Amerchol) | 0,2 g | 0,1 g |
| Alcools en C₂₀-C₂₂ (Nafol 2022 EN vendu par Sasol) | 3 g | 3 g |
| Alcool oléique | 0,5 g | 0,5 g |
| Monoéthanolamide d'acide laurique | 2 g | 2 g |
| Alcool stéarylique oxyéthyléné à 2 moles d'oxyéthylène | 5 g | 5 g |
| Alcool stéarylique oxyéthyléné à 21 moles d'oxyéthylène | 3,8 g | 3,8 g |
| Acide oléique | 3 g | 3 g |
| Solution aqueuse à 60 % en poids de chlorure d'hexadiméthrine (Mexomère PO vendu par Chimex) | 2 g | 2 g |
| Solution aqueuse à 40 % en poids de polyquaternium-6 (Merquat 100 vendu par Ondéo) | 2 g | 2 g |
| TiO₂ | 0,2 g | 0,2 g |
| Hydroxypropylméthyl cellulose | 0,2 g | 0,1 g |
| Carbomer (Carbopol 980 vendu par Noveon) | - | 0,3 g |
| Réducteur, antioxydant, séquestrant, parfum | q.s. | q.s. |
| Eau déminéralisée q.s.p. | 100 g | 100 g |

### Protocole d'application

Chaque composition est diluée extemporanément, avec une fois et demie son poids d'eau oxygénée (pH voisin de 3) à 9 volumes pour la composition 3 et à 20 volumes pour la composition 4. Le mélange ainsi réalisé est une crème de bonne consistance qui s'applique facilement sur des cheveux gris, à 90 % de cheveux blancs, à raison de 10 g pour 1 g de cheveux, pendant 20 minutes. Les cheveux sont ensuite facilement rincés, lavés avec un shampooing standard et séchés.

La coloration capillaire est évaluée de manière visuelle. Les résultats obtenus sur les cheveux naturels gris, à 90 % de cheveux blancs, après traitement, sont les suivants :

| | **Nuance** |
|---|---|
| **Composition 3** | Rouge intense |
| **Composition 4** | Noir naturel |

Ces colorations possèdent de bonnes propriétés notamment en termes de sélectivité et de ténacité. Elles possèdent aussi une bonne intensité. Les compositions obtenues sont stables dans le temps.

## Revendications

1. Composition tinctoriale pour fibres kératiniques, comprenant, dans un milieu approprié pour la teinture :
A) un ou plusieurs éther(s) de cellulose cationique(s) comprenant de 4 000 à 10 000 motifs anhydroglucoses, lesdits motifs anhydroglucoses étant substitués par au moins :
(i) un substituant de formule [R₄R₅R₆R₉N⁺](X₂⁻), dans laquelle :
R₄ et R₅ représentent, indépendamment l'un de l'autre, un groupe méthyle ou éthyle,
R₆ représente un groupe alkyle, linéaire ou ramifié, en C₈-C₂₄ ou aralkyle dont la partie alkyle, linéaire ou ramifiée, est en C₈-C₂₄,
R₉ représente un groupe divalent permettant le rattachement au groupement anhydroglucose et choisi parmi -(B)_{q}-CH₂-CHOH-CH₂- et -CH₂CH₂-,
q désignant 0 ou 1,
B désignant un groupe divalent -(CH₂CH₂O)ₙ-,
n' un nombre entier allant de 1 à 100,
X₂⁻ représente un anion ; et
(ii) un substituant de formule [R₁R₂R₃R₈N⁺](X₁⁻), dans laquelle :
R₁, R₂, R₃ représentent, indépendamment l'un de l'autre, un groupe méthyle ou éthyle,
R₈ représente un groupe divalent permettant le rattachement au groupement anhydroglucose et choisi parmi -(A)_{P}-CH₂-CHOH-CH₂- et -CH₂CH₂-,
p désignant 0 ou 1,
A désignant un groupe divalent -(CH₂CH₂O)ₙ-,
n un nombre entier allant de 1 à 100,
X₁⁻ représente un anion ;
B) un ou plusieurs métasilicate(s) ; et
C) un ou plusieurs colorant(s) d'oxydation choisi(s) parmi les colorants d'oxydation benzéniques, hétérocycliques et naphtaléniques.

2. Composition tinctoriale selon la revendication 1, **caractérisée en ce que** l'éther de cellulose cationique est formé d'au moins un motif (IV) et d'au moins un des motifs (I), (II) ou (III) suivants : 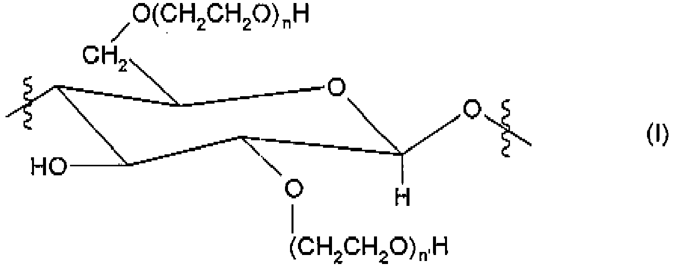 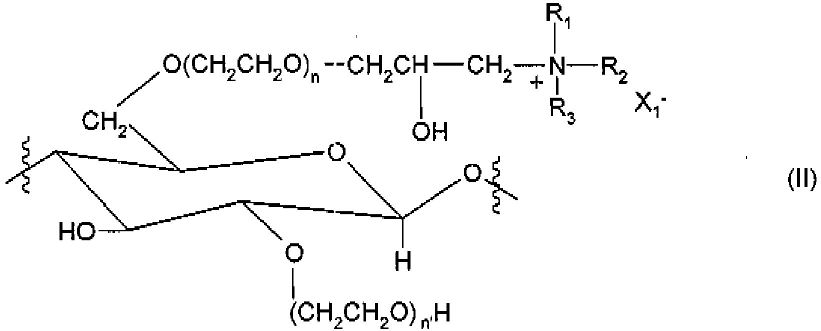 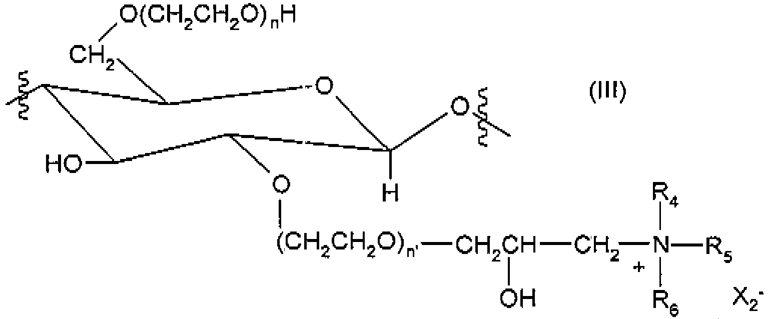 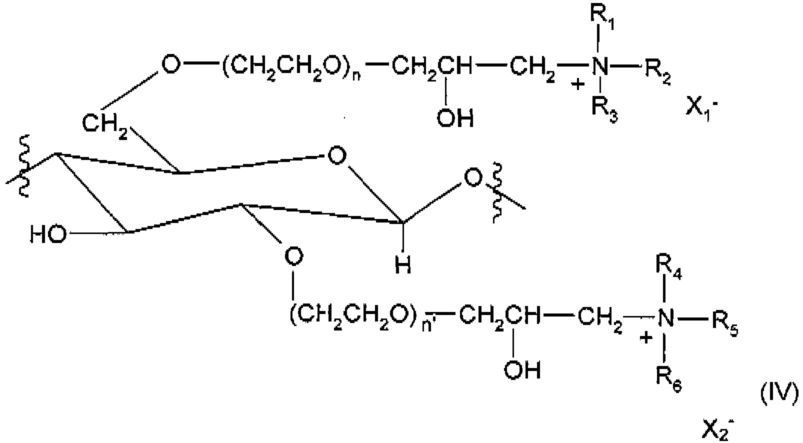 sous réserve que :
le nombre total des motifs (I) + (II) + (III) + (IV) soit compris entre 4000 et 10000 ;
le rapport des nombres de motifs [(III) + (IV)] / [(I) + (II) + (III) + (IV)] va de 0,0003 à 0,8 ;
le rapport des nombres de motifs [(II) + (IV)] / [(I) + (II) + (III) + (IV)] va de 0,02 à 0,9 ;
les nombres entiers n et n', indépendamment l'un de l'autre, vont de 0 à 5 ;
R₁, R₂, R₃, R₄ et R₅ représentent, indépendamment l'un de l'autre, un groupe méthyle ou éthyle ;
R₆ représente un groupement alkyle, linéaire ou ramifié, en C₈-C₂₄, ou un groupement aralkyle dont la partie alkyle, linéaire ou ramifiée, est en C₈-C₂₄ ;
X₁⁻ et X₂⁻ représentent des anions choisis, de préférence, indépendamment l'un de l'autre, parmi les ions phosphate, nitrate, sulfate et halogénure.

3. Composition tinctoriale selon la revendication 2, **caractérisée en ce que** l'éther de cellulose est tel que R₆ représente un groupement alkyl, linéaire ou ramifié, comprenant de 12 à 15 atomes de carbone, R₆ étant de préférence un groupement dodécyle linéaire.

4. Composition tinctoriale selon l'une des revendications 1 à 3, **caractérisée en ce que** la concentration en éther(s) de cellulose cationique(s) va de 0,01 à 10 % en poids, de préférence de 0,05 à 3 % en poids et de manière plus préférée de 0,1 à 1 % en poids, par rapport au poids total de la composition.

5. Composition tinctoriale selon l'une des revendications précédentes, **caractérisée en ce que** le métasilicate répond à la formule générale suivante :
(Y^{p+})ₙSiO₃²⁻
dans laquelle :
Y désigne un métal mono- ou divalent, de préférence un métal alcalin tel que par exemple Li, Na, K, ou alcalinoterreux tel que par exemple Ba, Mg, Ca, ou un groupe NH₄ ;
n = 1 ou 2, p = 1 ou 2, et en particulier, quand n = 1 alors p = 2 et quand n = 2 alors p = 1.

6. Composition tinctoriale selon l'une des revendications précédentes, **caractérisée en ce que** la concentration en métasilicate(s) va de 0,005 à 20 % en poids, de préférence de 0,1 à 10 % en poids, et de manière plus préférée, de 0,2 à 5 % en poids, par rapport au poids total de la composition.

7. Composition tinctoriale selon l'une des revendications précédentes, **caractérisée en ce que** le colorant d'oxydation est choisi parmi les bases benzéniques cationiques ou non, les bases hétérocycliques, les coupleurs benzéniques, les coupleurs hétérocycliques et les coupleurs naphtaléniques,

8. Composition tinctoriale selon la revendication 7, **caractérisée en ce que** le colorant d'oxydation est une base d'oxydation benzénique choisie parmi les para-phénylènediamines, les bis-phénylalkylènediamines, les para-aminophénols, les ortho-aminophénols, et leurs sels d'addition.

9. Composition tinctoriale selon la revendication 7, **caractérisée en ce que** le colorant d'oxydation est une base d'oxydation hétérocyclique choisie parmi les pyridines, les pyrimidines, les pyrazoles, les pyrazolopyrimidines condensées, les pyrazolotriazoles, les pyrazolotétrazoles, les pyrazolopyridazines, les pyrazolothiazoles, les pyrazoloimidazoles, les pyrazolobenzimidazoles, les pyrazoloquinolines, les aminopyrolidines, les aminopyrazolines, les mono- ou diaminotétraquinoléines, les diamino- ou triaminoquinoléines, les aminoindazoles, les diaminouraciles, les aminoindolénines, les hydrazones, la julolidine ou la lilolidine, ainsi que leurs dérivés et leurs sels d'addition.

10. Composition tinctoriale selon la revendication 7, **caractérisée en ce que** le colorant d'oxydation est un coupleur benzénique choisi parmi les méta-aminophénols, les méta-phénylènediamines, les méta-diphénols et leurs sels d'addition.

11. Composition tinctoriale selon la revendication 7, **caractérisée en ce que** le colorant d'oxydation est un coupleur hétérocyclique choisi parmi les coupleurs hétérocycliques azolés, les coupleurs pyridiniques, les thiophènes, les indolines, les indoles, les benzofuranes, les 8-amino-6-méthoxyquinoléines, les 4-hydroxyquinolones, les benzodioxoles, les hydroxybenzamides, le sésamol et ses dérivés, les benzomorpholines, ainsi que leurs sels d'addition.

12. Composition tinctoriale selon l'une des revendications précédentes, **caractérisée en ce que** la concentration en colorant(s) d'oxydation va de 0,005 à 15 % en poids, de préférence de 0,01 à 10 % en poids, et encore plus préférentiellement de 0,5 à 5 % en poids, par rapport au poids total de la composition.

13. Procédé de teinture d'oxydation des fibres kératiniques, **caractérisé en ce que** l'on applique sur les fibres une composition tinctoriale telle que définie dans l'une quelconque des revendications 1 à 12 en présence d'au moins un agent oxydant pendant un temps suffisant pour développer la couleur désirée.

14. Dispositif à plusieurs compartiments, **caractérisé en ce qu'**il comprend au moins un premier compartiment contenant une composition tinctoriale telle que définie dans l'une quelconque des revendications 1 à 12 et au moins un deuxième compartiment contenant au moins un agent oxydant.

15. Utilisation de la composition définie dans l'une des revendications 1 à 12 pour la teinture des fibres kératiniques, en particulier, des fibres kératiniques humaines telles que les cheveux.

## Patentansprüche

1. Färbezusammensetzung für Keratinfasern, die in einem für die Färbung geeigneten Medium Folgendes umfasst:
A) einen oder mehrere kationische Celluloseether mit 4000 bis 10.000 Anhydroglucose-Einheiten, wobei die Anhydroglucose-Einheiten durch mindestens:
(i) einen Substituenten der Formel [R₄R₅R₆R₉N⁺] (X₂⁻), in der:
R₄ und R₅ unabhängig voneinander für eine Methyl- oder Ethylgruppe stehen,
R₆ für eine lineare oder verzweigte C₈-C₂₄-Alkylgruppe oder eine Aralkylgruppe mit linearem oder verzweigtem C₈-C₂₄-Alkylteil steht,
R₉ für eine zweiwertige Gruppe steht, die die Verknüpfung mit der Anhydroglucose-Gruppe ermöglicht und aus -(B)_{q}-CH₂-CHOH-CH₂- und -CH₂CH₂- ausgewählt ist,
wobei q 0 oder 1 bedeutet,
wobei B eine zweiwertige Gruppe -(CH₂CH₂O)_{n'}- bedeutet,
wobei n' eine ganze Zahl im Bereich von 1 bis 100 ist,
X₂⁻ für ein Anion steht; und
(ii) einen Substituenten der Formel [R₁R₂R₃R₈N⁺] (X₁⁻), in der:
R₁, R₂ und R₃ unabhängig voneinander für eine Methyl- oder Ethylgruppe stehen,
R₈ für eine zweiwertige Gruppe steht, die die Verknüpfung mit der Anhydroglucose-Gruppe ermöglicht und aus - (A)ₚ-CH₂-CHOH-CH₂- und -CH₂CH₂- ausgewählt ist,
wobei p 0 oder 1 bedeutet,
wobei A eine zweiwertige Gruppe -(CH₂CH₂O)ₙ- bedeutet,
wobei n eine ganze Zahl im Bereich von 1 bis 100 ist,
X₁⁻ für ein Anion steht;
substituiert sind;
B) ein oder mehrere Metasilikate und
C) einen oder mehrere Oxidationsfarbstoffe, die aus Benzol-Oxidationsfarbstoffen, heterocyclischen Oxidationsfarbstoffen und Naphthalin-Oxidationsfarbstoffen ausgewählt sind.

2. Färbezusammensetzung nach Anspruch 1, **dadurch gekennzeichnet, dass** der kationische Celluloseether aus mindestens einer Einheit (IV) und mindestens einer der folgenden Einheiten (I), (II) oder (III) besteht: 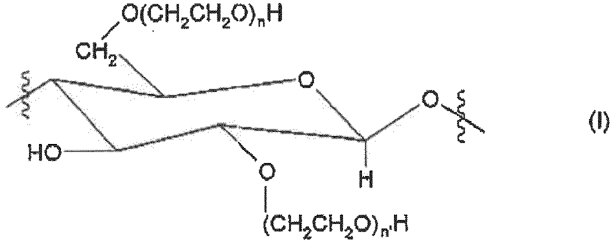 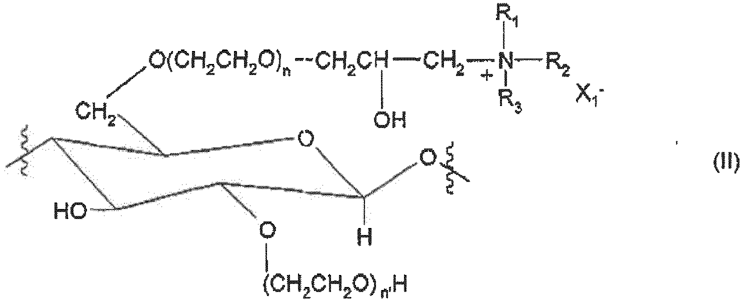 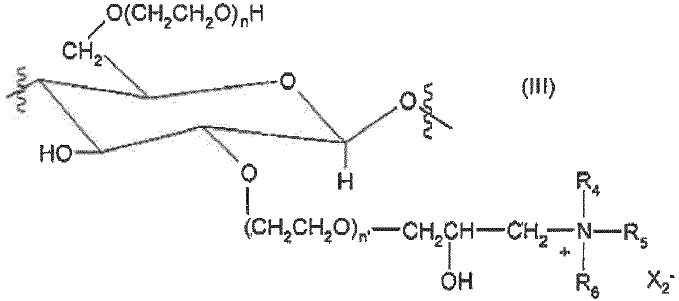 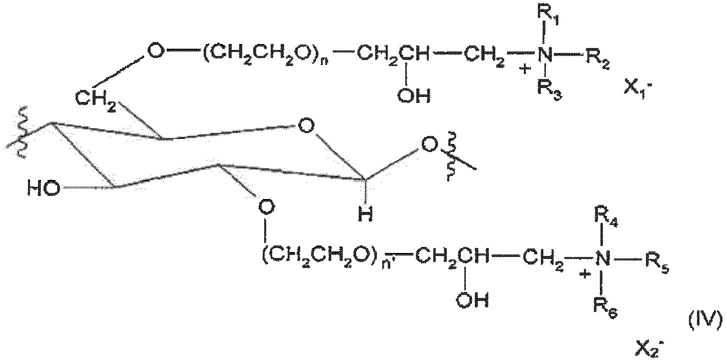 mit den Maßgaben, dass:
die Gesamtzahl der Einheiten (I) + (II) + (III) + (IV) zwischen 4000 und 10.000 liegt;
das Verhältnis der Zahl der Einheiten [(III) + (IV)]/[(I) + (II) + (III) + (IV)] im Bereich von 0,0003 bis 0,8 liegt;
das Verhältnis der Zahl der Einheiten [(II) + (IV)]/[(I) + (II) + (III) + (IV)] im Bereich von 0,02 bis 0,9 liegt;
die ganzen Zahlen n und n' unabhängig voneinander im Bereich von 0 bis 5 liegen;
R₁, R₂, R₃, R₄ und R₅ unabhängig voneinander für eine Methyl- oder Ethylgruppe stehen;
R₆ für eine lineare oder verzweigte C₈-C₂₄-Alkylgruppe oder eine Aralkylgruppe mit linearem oder verzweigtem C₈-C₂₄-Alkylteil steht;
X₁⁻ und X₂⁻ für Anionen stehen, die vorzugsweise unabhängig voneinander aus Phosphat-, Nitrat-, Sulfat- und Halogenidionen ausgewählt sind.

3. Färbezusammensetzung nach Anspruch 2, **dadurch gekennzeichnet, dass** der Celluloseether so beschaffen ist, dass R₆ für eine lineare oder verzweigte Alkylgruppe mit 12 bis 15 Kohlenstoffatomen steht, wobei R₆ vorzugsweise eine lineare Dodecylgruppe ist.

4. Färbezusammensetzung nach einem der Ansprüche 1 bis 3, **dadurch gekennzeichnet, dass** die Konzentration von kationischem (kationischen) Celluloseether(n) im Bereich von 0,01 bis 10 Gew.-%, vorzugsweise von 0,05 bis 3 Gew.-% und weiter bevorzugt von 0,1 bis 1 Gew.-%, bezogen auf das Gesamtgewicht der Zusammensetzung, liegt.

5. Färbezusammensetzung nach einem der vorhergehenden Ansprüche, **dadurch gekennzeichnet, dass** das Metasilikat der folgenden allgemeinen Formel entspricht:
(Y^{p+})ₙSiO₃²⁻,
in der:
y ein ein- oder zweiwertiges Metall, vorzugsweise ein Alkalimetall wie beispielsweise Li, Na oder K, oder ein Erdalkalimetall wie beispielsweise Ba, Mg oder Ca, oder eine NH₄-Gruppe bedeutet;
n = 1 oder 2, p = 1 oder 2, und insbesondere, wenn n = 1, dann p = 2, und wenn n = 2, dann p = 1.

6. Färbezusammensetzung nach einem der vorhergehenden Ansprüche, **dadurch gekennzeichnet, dass** die Konzentration von Metasilikat(en) im Bereich von 0,005 bis 20 Gew.-%, vorzugsweise von 0,1 bis 10 Gew.-% und weiter bevorzugt von 0,2 bis 5 Gew.-%, bezogen auf das Gesamtgewicht der Zusammensetzung, liegt.

7. Färbezusammensetzung nach einem der vorhergehenden Ansprüche, **dadurch gekennzeichnet, dass** der Oxidationsfarbstoff aus kationischen oder nicht kationischen Benzol-Basen, heterocyclischen Basen, Benzol-Kupplern, heterocyclischen Kupplern und Naphthalin-Kupplern ausgewählt ist.

8. Färbezusammensetzung nach Anspruch 7, **dadurch gekennzeichnet, dass** es sich bei dem Oxidationsfarbstoff um eine Benzol-Oxidationsbase handelt, die aus para-Phenylendiaminen, Bisphenylalkylendiaminen, para-Aminophenolen, ortho-Aminophenolen und Additionssalzen davon ausgewählt ist.

9. Färbezusammensetzung nach Anspruch 7, **dadurch gekennzeichnet, dass** es sich bei dem Oxidationsfarbstoff um eine heterocyclische Oxidationsbase handelt, die aus Pyridinen, Pyrimidinen, Pyrazolen, kondensierten Pyrazolopyrimidinen, Pyrazolotriazolen, Pyrazolotetrazolen, Pyrazolopyridazinen, Pyrazolothiazolen, Pyrazoloimidazolen, Pyrazolobenzimidazolen, Pyrazolochinolinen, Aminopyrrolidinen, Aminopyrazolinen, Mono- oder Diaminotetrachinolinen, Diamino- oder Triaminochinolinen, Aminoindazolen, Diaminouracilen, Aminoindoleninen, Hydrazonen, Julolidin oder Lilolidin sowie Derivaten davon und Additionssalzen davon ausgewählt ist.

10. Färbezusammensetzung nach Anspruch 7, **dadurch gekennzeichnet, dass** es sich bei dem Oxidationsfarbstoff um einen Benzol-Kuppler handelt, der aus meta-Aminophenolen, meta-Phenylendiaminen, meta-Diphenolen und Additionssalzen davon ausgewählt ist.

11. Färbezusammensetzung nach Anspruch 7, **dadurch gekennzeichnet, dass** es sich bei dem Oxidationsfarbstoff um einen heterocyclischen Kuppler handelt, der aus heterocyclischen Azol-Kupplern, Pyridin-Kupplern, Thiophenen, Indolinen, Indolen, Benzofuranen, 8-Amino-6-methoxychinolinen, 4-Hydroxychinolonen, Benzodioxolen, Hydroxybenzamiden, Sesamol und Derivaten davon, Benzomorpholinen sowie Additionssalzen davon ausgewählt ist.

12. Färbezusammensetzung nach einem der vorhergehenden Ansprüche, **dadurch gekennzeichnet, dass** die Konzentration an Oxidationsfarbstoff(en) im Bereich von 0,005 bis 15 Gew.-%, vorzugsweise von 0,01 bis 10 Gew.-% und weiter bevorzugt von 0,5 bis 5 Gew.-%, bezogen auf das Gesamtgewicht der Zusammensetzung, liegt.

13. Verfahren zum Oxidationsfärben von Keratinfasern, **dadurch gekennzeichnet, dass** man auf die Fasern eine Färbezusammensetzung gemäß einem der Ansprüche 1 bis 12 in Gegenwart mindestens eines Oxidationsmittels über einen zur Entwicklung der gewünschten Farbe ausreichenden Zeitraum aufbringt.

14. Vorrichtung mit mehreren Kompartimenten, **dadurch gekennzeichnet, dass** sie mindestens ein erstes Kompartiment, das eine Färbezusammensetzung gemäß einem der Ansprüche 1 bis 12 enthält, und mindestens ein zweites Kompartiment, das mindestens ein Oxidationsmittel enthält, umfasst.

15. Verwendung der Zusammensetzung gemäß einem der Ansprüche 1 bis 12 zum Färben von Keratinfasern, insbesondere menschlichen Keratinfasern wie dem Haar.

## Claims

1. Dyeing composition for keratinous fibers comprising, in a medium appropriate for dyeing:
A) one or more cationic cellulose ether(s) comprising from 4000 to 10 000 anhydroglucose units, said anhydroglucose units being substituted by at least:
(i) one substituent of formula [R₄R₅R₆R₉N⁺] (X₂⁻), in which:
R₄ and R₅ represent, independently of one another, a methyl or ethyl group,
R₆ represents a linear or branched C₈-C₂₄ alkyl group or an aralkyl group, the linear or branched alkyl part of which is a C₈-C₂₄ alkyl group,
R₉ represents a divalent group which makes possible the uniting with the anhydroglucose group and which is chosen from -(B)_{q}-CH₂-CHOH-CH₂- and -CH₂CH₂-,
q denoting 0 or 1,
B denoting a divalent group -(CH₂CH₂O)_{n'}-,
n' being an integer ranging from 1 to 100,
X₂⁻ represents an anion; and
(ii) a substituent of formula [R₁R₂R₃R₈N⁺] (X₁⁻), in which:
R₁, R₂ and R₃ represent, independently of one another, a methyl or ethyl group,
R₈ represents a divalent group which makes possible the uniting with the anhydroglucose group and which is chosen from - (A)ₚ-CH₂-CHOH-CH₂- and -CH₂CH₂-,
p denoting 0 or 1,
A denoting a divalent group -(CH₂CH₂O)ₙ-,
n being an integer ranging from 1 to 100,
X₁⁻ represents an anion;
B) one or more metasilicate(s); and
C) one or more oxidation dye(s) chosen from benzene, heterocyclic and naphthalene oxidation dyes.

2. Dyeing composition according to Claim 1, **characterized in that** the cationic cellulose ether is formed of at least one unit (IV) and of at least one of the units (I), (II) or (III) below: 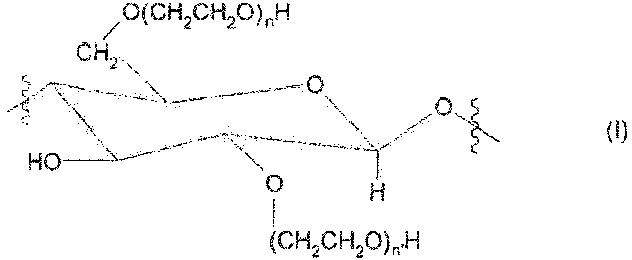 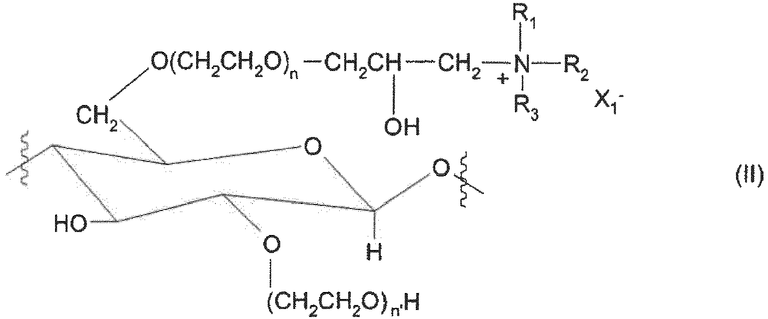 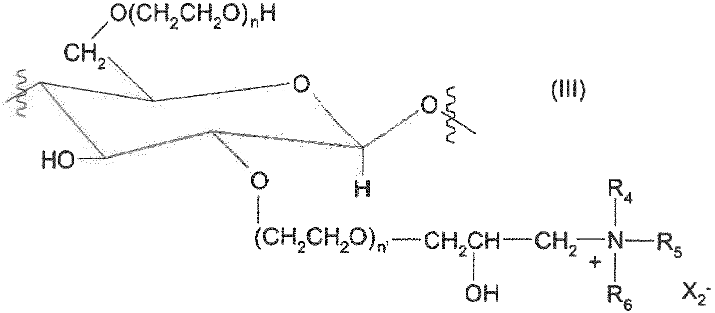 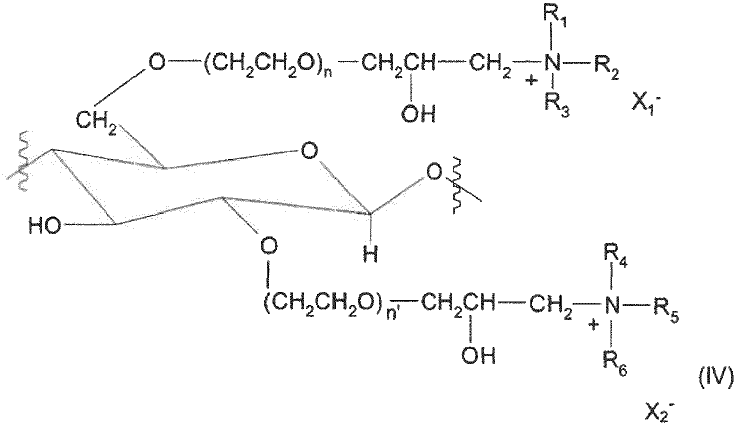 with the proviso that:
the total number of the units (I) + (II) + (III) + (IV) is between 4000 and 10 000;
the ratio of the numbers of units [(III) + (IV)]/[(I) + (II) + (III) + (IV)] ranges from 0.0003 to 0.8;
the ratio of the numbers of units [(II) + (IV)]/[(I) + (II) + (III) + (IV)] ranges from 0.02 to 0.9;
the integers n and n' range, independently of one another, from 0 to 5;
R₁, R₂, R₃, R₄ and R₅ represent, independently of one another, a methyl or ethyl group;
R₆ represents a linear or branched C₈-C₂₄ alkyl group or an aralkyl group, the linear or branched alkyl part of which is a C₈-C₂₄ alkyl group;
X₁⁻ and X₂⁻ represent anions preferably chosen, independently of one another, from phosphate, nitrate, sulfate and halide ions.

3. Dyeing composition according to Claim 2, **characterized in that** the cellulose ether is such that R₆ represents a linear or branched alkyl group comprising from 12 to 15 carbon atoms, R₆ preferably being a linear dodecyl group.

4. Dyeing composition according to one of Claims 1 to 3, **characterized in that** the concentration of cationic cellulose ether(s) ranges from 0.01 to 10% by weight, preferably from 0.05 to 3% by weight and more preferably from 0.1 to 1% by weight, with respect to the total weight of the composition.

5. Dyeing composition according to one of the preceding claims, **characterized in that** the metasilicate corresponds to the following general formula:
(Y^{p+})ₙSiO₃²⁻
in which:
Y denotes a mono- or divalent metal, preferably an alkali metal, such as, for example, Li, Na or K, or an alkaline earth metal, such as, for example, Ba, Mg or Ca, or an NH₄ group;
n = 1 or 2, p = 1 or 2, and in particular, when n = 1, then p = 2 and, when n = 2, then p = 1.

6. Dyeing composition according to one of the preceding claims, **characterized in that** the concentration of metasilicate(s) ranges from 0.005 to 20% by weight, preferably from 0.1 to 10% by weight and more preferably from 0.2 to 5% by weight, with respect to the total weight of the composition.

7. Dyeing composition according to one of the preceding claims, **characterized in that** the oxidation dye is chosen from cationic or noncationic benzene bases, heterocyclic bases, benzene couplers, heterocyclic couplers and naphthalene couplers.

8. Dyeing composition according to Claim 7, **characterized in that** the oxidation dye is a benzene oxidation base chosen from para-phenylenediamines, bis-phenylalkylenediamines, para-aminophenols, ortho-aminophenols and their addition salts.

9. Dyeing composition according to Claim 7, **characterized in that** the oxidation dye is a heterocyclic oxidation base chosen from pyridines, pyrimidines, pyrazoles, fused pyrazolopyrimidines, pyrazolotriazoles, pyrazolotetrazoles, pyrazolopyridazines, pyrazolothiazoles, pyrazoloimidazoles, pyrazolobenzimidazoles, pyrazoloquinolines, amino-pyrrolidines, aminopyrazolines, mono- or diaminotetra-quinolines, diamino- or triaminoquinolines, aminoindazoles, diaminouracils, aminoindolenines, hydrazones, julolidine or lilolidine, and their derivatives and their addition salts.

10. Dyeing composition according to Claim 7, **characterized in that** the oxidation dye is a benzene coupler chosen from meta-aminophenols, meta-phenylenediamines, meta-diphenols and their addition salts.

11. Dyeing composition according to Claim 7, **characterized in that** the oxidation dye is a heterocyclic coupler chosen from azole heterocyclic couplers, pyridine couplers, thiophenes, indolines, indoles, benzofurans, 8-amino-6-methoxyquinolines, 4-hydroxyquinolones, benzodioxoles, hydroxybenzamides, sesamol and its derivatives, benzomorpholines and their addition salts.

12. Dyeing composition according to one of the preceding claims, **characterized in that** the concentration of oxidation dye(s) ranges from 0.005 to 15% by weight, preferably from 0.01 to 10% by weight and more preferably still from 0.5 to 5% by weight, with respect to the total weight of the composition.

13. Method for the oxidation dyeing of keratinous fibers, **characterized in that** a dyeing composition as defined in any one of Claims 1 to 12 is applied to the fibers in the presence of at least one oxidizing agent for a time sufficient to develop the desired color.

14. Multicompartment device, **characterized in that** it comprises at least one first compartment comprising a dyeing composition as defined in any one of Claims 1 to 12 and at least one second compartment comprising at least one oxidizing agent.

15. Use of the composition defined in one of Claims 1 to 12 for the dyeing of keratinous fibers, in particular human keratinous fibers, such as the hair.
